# EUROPEAN PATENT APPLICATION

(11) **EP 4 386 000 A1**
(43) Date of publication of application: **19.06.2024**
(21) Application number: 22855436.6
(22) Date of filing: 09.08.2022
(51) Int. Cl.: C07K 16/18, C12N 15/13, A61K 39/395, A61P 37/06

(54) **RECOMBINANT ANTI-HUMAN-CD25 ANTIBODY AND USE THEREOF**

(30) Priority: 09.08.2021 CN 202110907431
(71) Applicant: Nanjing Novoacine Bio-Technology Co., Ltd., Nanjing City, Jiangsu 210032 (CN)
(72) Inventor: XIONG, Xinhui, Nanjing City, Jiangsu 210032 (CN); ZHANG, Tao, Nanjing City, Jiangsu 210032 (CN); ZHONG, Kai, Nanjing City, Jiangsu 210032 (CN); WU, Wei, Nanjing City, Jiangsu 210032 (CN); HUANG, Qianhui, Nanjing City, Jiangsu 210032 (CN); YE, Yi, Nanjing City, Jiangsu 210032 (CN); LI, Xing, Nanjing City, Jiangsu 210032 (CN); PAN, Hong, Nanjing City, Jiangsu 210032 (CN)
(74) Representative: Schiweck Weinzierl Koch Patentanwälte Partnerschaft mbB
(86) International application number: PCT/CN2022/111165
(87) International publication number: WO 2023/016455

(57) **Abstract**

An anti-human-CD25 antibody molecule or an antigen-binding fragment thereof. Compared with an existing anti-CD25 antibody, the provided antibody is non-IL-2 inhibitory, and can not only eliminate Treg, but can also correct a tumor microenvironment and increase the proportion and killing ability of Teff. Further provided is the use of the antibody molecule or the antigen-binding fragment in the preparation of a drug for treating solid tumors and hematological tumors.

## Description

### CROSS-REFERENCE TO RELATED APPLICATION(S)

The present patent application claims priority to and the benefit of Chinese patent application No. 202110907431.6 filed on August 9, 2021, which is incorporated herein by reference in its entirety.

### TECHNICAL FIELD

The present invention belongs to the field of antibody medicine, and particularly relates to an antibody against human CD25 and use of the antibody in preparing medicaments.

### BACKGROUND OF THE INVENTION

Cancer immunotherapy is to enhance the anti-tumor immunity in tumor microenvironment through mobilizing the immune system of a body, so as to control and kill tumor cells. However, malignant tumors (particularly solid tumors) can escape immune surveillance through a variety of mechanisms mediated by the tumor cells themselves and by components in the tumor microenvironment. In this case, it needs to be considered how the complicated interactions between regulatory T cell (Treg) and effector T cell (Teff) populations determine the outcome of the tumor-specific immune response. In a tumor-specific immune response, Tregs can promote the establishment and maintenance of peripheral tolerance and play a vital role in mediating immune homeostasis; however, their effects are more complex in the context of cancers, and the imbalance in Teff cells versus Treg cells (i.e., an imbalance in Teff/Treg ratio) due to tumor infiltration by Tregs has been generally recognized as a key factor (Nat Rev Cancer 2014, 14(11): 722-35.).

As cancer cells express both self- and tumor-associated antigens, the presence of Tregs which seek to dampen effector cell response can contribute to tumor progression. The infiltration of Tregs in established tumors represents one of the main obstacles to effective anti-tumor responses and to the treatment of cancers in general. Tregs employing suppressive mechanisms are thought to contribute significantly to the limitations or even failure of current therapies, especially immunotherapies that rely on the induction or enhancement of anti-tumor responses (Anticancer Res 2012, 32(3): 997-1003.). Tumor infiltration of Tregs, one of the major obstacles to tumor immunotherapy success, is also associated with several poorly-prognostic human cancers (Shang B et al, 2015, Sci Rep. 5: 15179). Treg cells contribute to the early establishment and progression of tumors in murine models, and that their absence results in delay of tumor progression. In humans, high tumor infiltration by Treg cells and, more importantly, a low ratio of effector T (Teff) cells to Treg cells, is associated with poor outcomes in multiple solid cancers. Conversely, a high Teff/Treg cell ratio is associated with favorable responses to immunotherapy in both humans and mice. To date, most studies support the notion that targeting Treg cells, either by depletion or functional modulation, may offer significant therapeutic benefit, particularly in combination with other immune modulatory interventions such as vaccines and checkpoint blockade (Immunity 2017, 46(4): 577-586.).

Defining appropriate targets for selective interference with Treg cells is therefore a critical step in the development of effective therapies. In this regard, CD25 was proposed as a target. CD25 is also known as the interleukin-2 high-affinity receptor α chain (IL-2Rα), and was the first surface marker used to identify and isolate Treg cells prior to the discovery of their master regulator, transcription factor forkhead box P3 (FoxP3). CD25 is also the most extensively studied target for mediating Treg cell depletion. Whereas CD25 is constitutively expressed on Treg cells and absent on naive Teff cells, transient upregulation has been described upon activation of Teff cells, as observed in in vitro studies. Basiliximab and Daclizumab that have been on the market for many years are anti-human CD25 antibodies that inhibit IL-2 binding to CD25. Basiliximab is a chimeric mouse-human CD25 antibody currently approved for graft versus host disease, and Daclizumab is a humanized CD25 antibody approved for the treatment of multiple sclerosis. A few other anti-CD25 antibodies (e.g., 7D4, 2E4, MA251, 7G7B6, etc.) although bind to CD25, do not inhibit the binding of IL-2 to CD25.

A CD25-targeted antibody with antibody-dependent cell-mediated cytotoxicity (ADCC) effect can effectively deplete Tregs in a tumor microenvironment and regulate the Teff/Treg ratio. In addition, CD25 is the alpha subunit of the receptor for IL-2 and IL-2 is a key cytokine for Teff responses. Therefore, antibodies targeting CD25 on the one hand can effectively deplete Tregs in the tumor microenvironment and on the other hand do not inhibit the IL-2 signaling through CD25, so that not only Treg cells can be removed mechanically, but also IL-2 can be allowed to stimulate Teff cells, and in turn a strong anti-tumor effect can be exerted.

Therefore, the development of an anti-CD25 antibody which is non-IL-2 blocking helps to correct tumor microenvironment, promotes the immune system to deplete cancer cells, and has important clinical significance for the treatment of cancer patients at present.

### SUMMARY OF THE INVENTION

The technical problem to be solved by the present invention is to obtain a new anti-CD25 antibody which is non-IL-2 blocking through hybridoma screening, humanization, recombination and other techniques.

For the above technical problem, an object of the present invention is to provide an anti-CD25 antibody molecule or antigen binding fragment thereof which, compared with existing anti-CD25 antibodies, is non-IL-2 blocking, so can not only deplete Tregs, thereby increasing the proportion of Teffs, but also allow IL-2 to stimulate Teff cells, thereby increasing the killing ability of Teff cells and correcting the tumor microenvironment.

"Fragment" of an antibody as described in the present disclosure encompasses various functional or active fragments of the antibody, e.g., an antigen-binding portion thereof, such as Fab, F(ab')2, or scFv.

"At least 75% identity" as described in the present disclosure is any percent identity greater than or equal to 75%, such as at least 80%, preferably at least 85%, more preferably at least 90%, further preferably at least 91%, 92%, 93%, 94%, 95%, 96%, 97%, 98%, or even 99% identity.

In one aspect, the present disclosure provides an antibody molecule or antigen-binding fragment thereof comprising a heavy chain variable region (VH) and a light chain variable region (VL), wherein the heavy chain variable region (VH) and the light chain variable region (VL) comprise heavy chain CDRs and light chain CDRs selected from the group consisting of the following combinations of CDRs:
(1) H-CDR1, H-CDR2, and H-CDR3 as shown in SEQ ID NOs: 63, 64 and 65 respectively; and L-CDR1, L-CDR2, L-CDR3 as shown in SEQ ID NOs: 82, 83, and 84 respectively;
(2) H-CDR1, H-CDR2, and H-CDR3 as shown in SEQ ID NOs: 63, 64, and 65 respectively; and L-CDR1, L-CDR2, L-CDR3 as shown in SEQ ID NOs: 85, 83, and 84 respectively;
(3) H-CDR1, H-CDR2, and H-CDR3 as shown in SEQ ID NOs: 63, 66, and 65 respectively; and L-CDR1, L-CDR2, L-CDR3 as shown in SEQ ID NOs: 86, 83, and 84 respectively;
(4) H-CDR1, H-CDR2, and H-CDR3 as shown in SEQ ID NOs: 63, 66, and 65 respectively; and L-CDR1, L-CDR2, L-CDR3 as shown in SEQ ID NOs: 87, 83, and 84 respectively;
(5) H-CDR1, H-CDR2, and H-CDR3 as shown in SEQ ID NOs: 63, 67, and 68 respectively; and L-CDR1, L-CDR2, L-CDR3 as shown in SEQ ID NOs: 82, 83, and 88 respectively;
(6) H-CDR1, H-CDR2, and H-CDR3 as shown in SEQ ID NOs: 63, 67, and 68 respectively; and L-CDR1, L-CDR2, L-CDR3 as shown in SEQ ID NOs: 85, 83, and 88 respectively;
(7) H-CDR1, H-CDR2, and H-CDR3 as shown in SEQ ID NOs: 69, 70, and 71 respectively; and L-CDR1, L-CDR2, L-CDR3 as shown in SEQ ID NOs: 89, 90, and 91 respectively;
(8) H-CDR1, H-CDR2, and H-CDR3 as shown in SEQ ID NOs: 69, 70, and 71 respectively; and L-CDR1, L-CDR2, L-CDR3 as shown in SEQ ID NOs: 92, 90, and 91 respectively;
(9) H-CDR1, H-CDR2, and H-CDR3 as shown in SEQ ID NOs: 69, 70, and 71 respectively; and L-CDR1, L-CDR2, L-CDR3 as shown in SEQ ID NOs: 92, 93, and 91 respectively;
(10) H-CDR1, H-CDR2, and H-CDR3 as shown in SEQ ID NOs: 63, 72, and 68 respectively; and L-CDR1, L-CDR2, L-CDR3 as shown in SEQ ID NOs: 82, 83, and 84 respectively;
(11) H-CDR1, H-CDR2, and H-CDR3 as shown in SEQ ID NOs: 63, 72, and 68 respectively; and L-CDR1, L-CDR2, L-CDR3 as shown in SEQ ID NOs: 85, 83, and 84 respectively;
(12) H-CDR1, H-CDR2, and H-CDR3 as shown in SEQ ID NOs: 73, 74, and 68 respectively; and L-CDR1, L-CDR2, L-CDR3 as shown in SEQ ID NOs: 82, 83, and 84 respectively;
(13) H-CDR1, H-CDR2, and H-CDR3 as shown in SEQ ID NOs: 73, 74, and 68 respectively; and L-CDR1, L-CDR2, L-CDR3 as shown in SEQ ID NOs: 85, 83, and 84 respectively;
(14) H-CDR1, H-CDR2, and H-CDR3 as shown in SEQ ID NOs: 75, 76, and 77 respectively; and L-CDR1, L-CDR2, L-CDR3 as shown in SEQ ID NOs: 94, 95, and 96 respectively;
(15) H-CDR1, H-CDR2, and H-CDR3 as shown in SEQ ID NOs: 75, 76, and 77 respectively; and L-CDR1, L-CDR2, L-CDR3 as shown in SEQ ID NOs: 94, 95, and 97 respectively;
(16) H-CDR1, H-CDR2, and H-CDR3 as shown in SEQ ID NOs: 75, 76, and 77 respectively; and L-CDR1, L-CDR2, L-CDR3 as shown in SEQ ID NOs: 94, 95, and 98 respectively;
(17) H-CDR1, H-CDR2, and H-CDR3 as shown in SEQ ID NOs: 78, 79, and 80 respectively; and L-CDR1, L-CDR2, L-CDR3 as shown in SEQ ID NOs: 99, 100, and 101 respectively;
(18) H-CDR1, H-CDR2, and H-CDR3 as shown in SEQ ID NOs: 78, 81, and 80 respectively; and L-CDR1, L-CDR2, L-CDR3 as shown in SEQ ID NOs: 99, 100, and 101 respectively;
(19) H-CDR1, H-CDR2, and H-CDR3 as shown in SEQ ID NOs: 78, 79, and 80 respectively; and L-CDR1, L-CDR2, L-CDR3 as shown in SEQ ID NOs: 102, 100, and 101 respectively;
(20) H-CDR1, H-CDR2, and H-CDR3 as shown in SEQ ID NOs: 78, 81, and 80 respectively; and L-CDR1, L-CDR2, L-CDR3 as shown in SEQ ID NOs: 102, 100, and 101 respectively;
(21) H-CDR1, H-CDR2, and H-CDR3 as shown in SEQ ID NOs: 78, 79, and 80 respectively; and L-CDR1, L-CDR2, L-CDR3 as shown in SEQ ID NOs: 102, 103, and 101 respectively; and
(22) H-CDR1, H-CDR2, and H-CDR3 as shown in SEQ ID NOs: 78, 81, and 80 respectively; and L-CDR1, L-CDR2, L-CDR3 as shown in SEQ ID NOs: 102, 103, and 101 respectively.

The antibody molecule or antigen-binding fragment thereof provided in the present disclosure is an antibody molecule or antigen-binding fragment thereof against CD25. Preferably, the CD25 is human CD25.

In the antibody molecule or antigen-binding fragment thereof provided in the present disclosure, both the heavy chain variable region and the light chain variable region comprise the above CDRs and framework regions (FRs) therebetween, and those domains are arranged as follows: FR1-CDR1-FR2-CDR2-FR3-CDR3-FR4. According to the amino acid sequences of the heavy chain variable region and the light chain variable region provided by the present disclosure, the amino acid sequences of heavy chain CDRs and light chain CDRs contained therein can be determined conventionally by those skilled in the art. For example, according to the particular embodiments of the present disclosure, the CDRs in the variable region amino acid sequences can be determined using Kabat, IMGT, ABM and Chotia numbering schemes. The obtained heavy chain CDRs and light chain CDRs determined by other methods known in the art and their combinations are also covered by the scope of the present disclosure.

Preferably, the heavy chain variable region comprises an amino acid sequence as shown in any one of SEQ ID NOs: 1-25 or an amino acid sequence having at least 75% identity to the amino acid sequence as shown in any one of SEQ ID NOs: 1-25; and, the light chain variable region comprises an amino acid sequence as shown in any one of SEQ ID NOs: 26-62 or an amino acid sequence having at least 75% identity to the amino acid sequence as shown in any one of SEQ ID NOs: 26-62.

Further preferably, the heavy chain variable region and the light chain variable region comprised in the antibody molecule or antigen-binding fragment thereof comprise amino acid sequences selected from the group consisting of the following combinations:
(1) an amino acid sequence as shown in SEQ ID NO: 1 or an amino acid sequence having at least 75% identity to the amino acid sequence as shown in SEQ ID NO: 1; and, an amino acid sequence as shown in SEQ ID NO: 26 or an amino acid sequence having at least 75% identity to the amino acid sequence as shown in SEQ ID NO: 26;
(2) an amino acid sequence as shown in SEQ ID NO: 2 or an amino acid sequence having at least 75% identity to the amino acid sequence as shown in SEQ ID NO: 2; and, an amino acid sequence as shown in SEQ ID NO: 27 or an amino acid sequence having at least 75% identity to the amino acid sequence as shown in SEQ ID NO: 27;
(3) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 27 or an amino acid sequence having at least 75% identity to the amino acid sequence as shown in SEQ ID NO: 27;
(4) an amino acid sequence as shown in SEQ ID NO: 2 or an amino acid sequence having at least 75% identity to the amino acid sequence as shown in SEQ ID NO: 2; and, an amino acid sequence as shown in SEQ ID NO: 28 or an amino acid sequence having at least 75% identity to the amino acid sequence as shown in SEQ ID NO: 28;
(5) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 28 or an amino acid sequence having at least 75% identity to the amino acid sequence as shown in SEQ ID NO: 28;
(6) an amino acid sequence as shown in SEQ ID NO: 2 or an amino acid sequence having at least 75% identity to the amino acid sequence as shown in SEQ ID NO: 2; and, an amino acid sequence as shown in SEQ ID NO: 29 or an amino acid sequence having at least 75% identity to the amino acid sequence as shown in SEQ ID NO: 29;
(7) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 29 or an amino acid sequence having at least 75% identity to the amino acid sequence as shown in SEQ ID NO: 29;
(8) an amino acid sequence as shown in SEQ ID NO: 4 or an amino acid sequence having at least 75% identity to the amino acid sequence as shown in SEQ ID NO: 4; and, an amino acid sequence as shown in SEQ ID NO: 30 or an amino acid sequence having at least 75% identity to the amino acid sequence as shown in SEQ ID NO: 30;
(9) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 31 or an amino acid sequence having at least 75% identity to the amino acid sequence as shown in SEQ ID NO: 31;
(10) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 31 or an amino acid sequence having at least 75% identity to the amino acid sequence as shown in SEQ ID NO: 31;
(11) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 32 or an amino acid sequence having at least 75% identity to the amino acid sequence as shown in SEQ ID NO: 32;
(12) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 32 or an amino acid sequence having at least 75% identity to the amino acid sequence as shown in SEQ ID NO: 32;
(13) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 33 or an amino acid sequence having at least 75% identity to the amino acid sequence as shown in SEQ ID NO: 33;
(14) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 33 or an amino acid sequence having at least 75% identity to the amino acid sequence as shown in SEQ ID NO: 33;
(15) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 34 or an amino acid sequence having at least 75% identity to the amino acid sequence as shown in SEQ ID NO: 34;
(16) an amino acid sequence as shown in SEQ ID NO: 7 or an amino acid sequence having at least 75% identity to the amino acid sequence as shown in SEQ ID NO: 7; and, an amino acid sequence as shown in SEQ ID NO: 35 or an amino acid sequence having at least 75% identity to the amino acid sequence as shown in SEQ ID NO: 35;
(17) an amino acid sequence as shown in SEQ ID NO: 8 or an amino acid sequence having at least 75% identity to the amino acid sequence as shown in SEQ ID NO: 8; and, an amino acid sequence as shown in SEQ ID NO: 36 or an amino acid sequence having at least 75% identity to the amino acid sequence as shown in SEQ ID NO: 36;
(18) an amino acid sequence as shown in SEQ ID NO: 9 or an amino acid sequence having at least 75% identity to the amino acid sequence as shown in SEQ ID NO: 9; and, an amino acid sequence as shown in SEQ ID NO: 36 or an amino acid sequence having at least 75% identity to the amino acid sequence as shown in SEQ ID NO: 36;
(19) an amino acid sequence as shown in SEQ ID NO: 8 or an amino acid sequence having at least 75% identity to the amino acid sequence as shown in SEQ ID NO: 8; and, an amino acid sequence as shown in SEQ ID NO: 37 or an amino acid sequence having at least 75% identity to the amino acid sequence as shown in SEQ ID NO: 37;
(20) an amino acid sequence as shown in SEQ ID NO: 9 or an amino acid sequence having at least 75% identity to the amino acid sequence as shown in SEQ ID NO: 9; and, an amino acid sequence as shown in SEQ ID NO: 37 or an amino acid sequence having at least 75% identity to the amino acid sequence as shown in SEQ ID NO: 37;
(21) an amino acid sequence as shown in SEQ ID NO: 8 or an amino acid sequence having at least 75% identity to the amino acid sequence as shown in SEQ ID NO: 8; and, an amino acid sequence as shown in SEQ ID NO: 38 or an amino acid sequence having at least 75% identity to the amino acid sequence as shown in SEQ ID NO: 38;
(22) an amino acid sequence as shown in SEQ ID NO: 9 or an amino acid sequence having at least 75% identity to the amino acid sequence as shown in SEQ ID NO: 9; and, an amino acid sequence as shown in SEQ ID NO: 38 or an amino acid sequence having at least 75% identity to the amino acid sequence as shown in SEQ ID NO: 38;
(23) an amino acid sequence as shown in SEQ ID NO: 10 or an amino acid sequence having at least 75% identity to the amino acid sequence as shown in SEQ ID NO: 10; and, an amino acid sequence as shown in SEQ ID NO: 39 or an amino acid sequence having at least 75% identity to the amino acid sequence as shown in SEQ ID NO: 39;
(24) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 40 or an amino acid sequence having at least 75% identity to the amino acid sequence as shown in SEQ ID NO: 40;
(25) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 40 or an amino acid sequence having at least 75% identity to the amino acid sequence as shown in SEQ ID NO: 40;
(26) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 41 or an amino acid sequence having at least 75% identity to the amino acid sequence as shown in SEQ ID NO: 41;
(27) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 41 or an amino acid sequence having at least 75% identity to the amino acid sequence as shown in SEQ ID NO: 41;
(28) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 42 or an amino acid sequence having at least 75% identity to the amino acid sequence as shown in SEQ ID NO: 42;
(29) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 42 or an amino acid sequence having at least 75% identity to the amino acid sequence as shown in SEQ ID NO: 42;
(30) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 43 or an amino acid sequence having at least 75% identity to the amino acid sequence as shown in SEQ ID NO: 43;
(31) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 43 or an amino acid sequence having at least 75% identity to the amino acid sequence as shown in SEQ ID NO: 43;
(32) an amino acid sequence as shown in SEQ ID NO: 13 or an amino acid sequence having at least 75% identity to the amino acid sequence as shown in SEQ ID NO: 13; and, an amino acid sequence as shown in SEQ ID NO: 44 or an amino acid sequence having at least 75% identity to the amino acid sequence as shown in SEQ ID NO: 44;
(33) an amino acid sequence as shown in SEQ ID NO: 14 or an amino acid sequence having at least 75% identity to the amino acid sequence as shown in SEQ ID NO: 14; and, an amino acid sequence as shown in SEQ ID NO: 45 or an amino acid sequence having at least 75% identity to the amino acid sequence as shown in SEQ ID NO: 45;
(34) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 45 or an amino acid sequence having at least 75% identity to the amino acid sequence as shown in SEQ ID NO: 45;
(35) an amino acid sequence as shown in SEQ ID NO: 14 or an amino acid sequence having at least 75% identity to the amino acid sequence as shown in SEQ ID NO: 14; and, an amino acid sequence as shown in SEQ ID NO: 46 or an amino acid sequence having at least 75% identity to the amino acid sequence as shown in SEQ ID NO: 46;
(36) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 46 or an amino acid sequence having at least 75% identity to the amino acid sequence as shown in SEQ ID NO: 46;
(37) an amino acid sequence as shown in SEQ ID NO: 14 or an amino acid sequence having at least 75% identity to the amino acid sequence as shown in SEQ ID NO: 14; and, an amino acid sequence as shown in SEQ ID NO: 47 or an amino acid sequence having at least 75% identity to the amino acid sequence as shown in SEQ ID NO: 47;
(38) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 47 or an amino acid sequence having at least 75% identity to the amino acid sequence as shown in SEQ ID NO: 47;
(39) an amino acid sequence as shown in SEQ ID NO: 16 or an amino acid sequence having at least 75% identity to the amino acid sequence as shown in SEQ ID NO: 16; and, an amino acid sequence as shown in SEQ ID NO: 48 or an amino acid sequence having at least 75% identity to the amino acid sequence as shown in SEQ ID NO: 48;
(40) an amino acid sequence as shown in SEQ ID NO: 17 or an amino acid sequence having at least 75% identity to the amino acid sequence as shown in SEQ ID NO: 17; and, an amino acid sequence as shown in SEQ ID NO: 49 or an amino acid sequence having at least 75% identity to the amino acid sequence as shown in SEQ ID NO: 49;
(41) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 49 or an amino acid sequence having at least 75% identity to the amino acid sequence as shown in SEQ ID NO: 49;
(42) an amino acid sequence as shown in SEQ ID NO: 17 or an amino acid sequence having at least 75% identity to the amino acid sequence as shown in SEQ ID NO: 17; and, an amino acid sequence as shown in SEQ ID NO: 50 or an amino acid sequence having at least 75% identity to the amino acid sequence as shown in SEQ ID NO: 50;
(43) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 50 or an amino acid sequence having at least 75% identity to the amino acid sequence as shown in SEQ ID NO: 50;
(44) an amino acid sequence as shown in SEQ ID NO: 17 or an amino acid sequence having at least 75% identity to the amino acid sequence as shown in SEQ ID NO: 17; and, an amino acid sequence as shown in SEQ ID NO: 51 or an amino acid sequence having at least 75% identity to the amino acid sequence as shown in SEQ ID NO: 51;
(45) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 51 or an amino acid sequence having at least 75% identity to the amino acid sequence as shown in SEQ ID NO: 51;
(46) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 52 or an amino acid sequence having at least 75% identity to the amino acid sequence as shown in SEQ ID NO: 52;
(47) an amino acid sequence as shown in SEQ ID NO: 19 or an amino acid sequence having at least 75% identity to the amino acid sequence as shown in SEQ ID NO: 19; and, an amino acid sequence as shown in SEQ ID NO: 53 or an amino acid sequence having at least 75% identity to the amino acid sequence as shown in SEQ ID NO: 53;
(48) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 54 or an amino acid sequence having at least 75% identity to the amino acid sequence as shown in SEQ ID NO: 54;
(49) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 54 or an amino acid sequence having at least 75% identity to the amino acid sequence as shown in SEQ ID NO: 54;
(50) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 55 or an amino acid sequence having at least 75% identity to the amino acid sequence as shown in SEQ ID NO: 55;
(51) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 55 or an amino acid sequence having at least 75% identity to the amino acid sequence as shown in SEQ ID NO: 55;
(52) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 56 or an amino acid sequence having at least 75% identity to the amino acid sequence as shown in SEQ ID NO: 56;
(53) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 56 or an amino acid sequence having at least 75% identity to the amino acid sequence as shown in SEQ ID NO: 56;
(54) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 57 or an amino acid sequence having at least 75% identity to the amino acid sequence as shown in SEQ ID NO: 57;
(55) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 57 or an amino acid sequence having at least 75% identity to the amino acid sequence as shown in SEQ ID NO: 57;
(56) an amino acid sequence as shown in SEQ ID NO: 22 or an amino acid sequence having at least 75% identity to the amino acid sequence as shown in SEQ ID NO: 22; and, an amino acid sequence as shown in SEQ ID NO: 58 or an amino acid sequence having at least 75% identity to the amino acid sequence as shown in SEQ ID NO: 58;
(57) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 59 or an amino acid sequence having at least 75% identity to the amino acid sequence as shown in SEQ ID NO: 59;
(58) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 59 or an amino acid sequence having at least 75% identity to the amino acid sequence as shown in SEQ ID NO: 59;
(59) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 59 or an amino acid sequence having at least 75% identity to the amino acid sequence as shown in SEQ ID NO: 59;
(60) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 60 or an amino acid sequence having at least 75% identity to the amino acid sequence as shown in SEQ ID NO: 60;
(61) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 60 or an amino acid sequence having at least 75% identity to the amino acid sequence as shown in SEQ ID NO: 60;
(62) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 60 or an amino acid sequence having at least 75% identity to the amino acid sequence as shown in SEQ ID NO: 60;
(63) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 61 or an amino acid sequence having at least 75% identity to the amino acid sequence as shown in SEQ ID NO: 61;
(64) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 61 or an amino acid sequence having at least 75% identity to the amino acid sequence as shown in SEQ ID NO: 61;
(65) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 61 or an amino acid sequence having at least 75% identity to the amino acid sequence as shown in SEQ ID NO: 61;
(66) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 62 or an amino acid sequence having at least 75% identity to the amino acid sequence as shown in SEQ ID NO: 62;
(67) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 62 or an amino acid sequence having at least 75% identity to the amino acid sequence as shown in SEQ ID NO: 62; and
(68) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 62 or an amino acid sequence having at least 75% identity to the amino acid sequence as shown in SEQ ID NO: 62.

With respect to the heavy chain variable region and the light chain variable region comprised in the anti-CD25 antibody or the antigen binding fragment thereof according to the present disclosure, the up to 25% difference due to the "at least 75% identity" may be present in any framework region in the heavy chain variable region or the light chain variable region. The difference may be resulted from amino acid deletion, addition or substitution at any position, and the substitution may be conservative substitution or non-conservative substitution.

The antibody molecule provided in the present disclosure is a murine antibody, a chimeric antibody, or a fully or partially humanized antibody; and, the antigen binding fragment is a half-antibody or a single-chain variable fragment (scFv), disulfide-stabilized Fv fragment (dsFv), (disulfide-stabilized Fv fragment)₂ (dsFv)₂, Fab fragment, Fab' fragment, F(ab')₂ fragment, or variable fragment (Fv) of the antibody molecule. Preferably, the antibody molecule is a monoclonal antibody or a single chain antibody. Preferably, the antibody molecule or antigen-binding fragment thereof further comprises a human or murine constant region, preferably a human or murine heavy chain constant region (CH) and/or a light chain constant region (CL). Preferably, the antibody molecule or antigen-binding fragment thereof comprises a heavy chain and a light chain. More preferably, the antibody molecule or antigen-binding fragment thereof comprises a heavy chain constant region of an IgG, IgA, IgM, IgD, or IgE and/or a light chain constant region of a x or λ type. Preferably, the antibody molecule or antigen-binding fragment thereof comprises a heavy chain constant region which is of human IgG1 or IgG4 subtype and comprises a light chain constant region which is of human kappa (κ) subtype. In general, the antibody molecule provided in the present disclosure is a monoclonal antibody, preferably a chimeric or humanized monoclonal antibody. Preferably, the antibody molecule comprises two identical heavy chains and two identical light chains linked via disulfide bonds.

According to one particular embodiment of the present disclosure, the heavy chain constant region of the antibody molecule comprises an amino acid sequence as shown in SEQ ID NO: 104 or an amino acid sequence having at least 75% identity to the amino acid sequence as shown in SEQ ID NO: 104; and the light chain constant region of the antibody molecule comprises an amino acid sequence as shown in SEQ ID NO: 105 or an amino acid sequence having at least 75% identity to the amino acid sequence as shown in SEQ ID NO: 105.

In another aspect, the present disclosure provides a nucleic acid molecule comprising a nucleotide sequence encoding a heavy chain CDR, a light chain CDR, a heavy chain variable region, a light chain variable region, a heavy chain or a light chain comprised in the antibody molecule or antigen binding fragment thereof according to the present disclosure.

In yet another aspect, the present disclosure provides a vector comprising the nucleic acid molecule according to the present disclosure. The vector can be a eukaryotic expression vector, a prokaryotic expression vector, an artificial chromosome, a phage vector and the like.

The vector or nucleic acid molecule of the present invention may be used to transform or transfect a host cell or in any way enter a host cell for antibody preservation or expression, etc. Thus, in a further aspect, the present disclosure provides a host cell comprising the nucleic acid molecule and/or the vector according to the present invention, or transformed or transfected with the nucleic acid molecule and/or the vector according to the present disclosure. The host cell may be any prokaryotic or eukaryotic cell, such as a bacterial or insect, fungus, plant or animal cell.

In still a further aspect, the present disclosure provides a composition comprising the antibody molecule or antigen-binding fragment thereof according to the present disclosure, the nucleic acid molecule according to the present disclosure, the vector according to the present disclosure, and/or the host cell according to the present disclosure. Preferably, the composition is a pharmaceutical composition, optionally comprising a pharmaceutically acceptable carrier, auxiliary material, or excipient.

In a further aspect, the present disclosure further provides use of the antibody molecule or antigen-binding fragment thereof, the nucleic acid molecule, the vector, the host cell, and/or the composition according to the present disclosure in the manufacture of a medicament for preventing, treating, and/or ameliorating a solid tumor or a hematological tumor.

In addition, the present disclosure further provides a method for preventing, treating and/or ameliorating a disease, comprising administering to a subject in need thereof a therapeutically effective amount of the antibody molecule or antigen-binding fragment thereof, the nucleic acid molecule, the vector, the host cell, and/or the composition according to the present disclosure, and optionally an additional drug or means. Preferably, the disease is a solid tumor or a hematological tumor. Additionally preferably, the subject is a mammal; preferably, the subject is a human.

The present disclosure also provides a method for detecting or diagnosing a disease, comprising contacting the antibody molecule or antigen-binding fragment thereof, the nucleic acid molecule, the vector, the host cell, and/or the composition according to the present disclosure with a sample from a subject. Preferably, the disease is a solid tumor or a hematological tumor. Additionally preferably, the subject is a mammal; preferably, the subject is a human.

In addition, the present disclosure further provides a kit comprising the antibody molecule or antigen-binding fragment thereof, the nucleic acid molecule, the vector, the host cell, and/or the composition according to the present disclosure. The kit may be used for detection or diagnosis, e.g. in the method for detecting or diagnosing a disease as described above.

The present disclosure provides a new antibody against CD25, which can bind to human CD25 with high affinity. And, as a non-IL-2 blocking antibody, it can not only deplete Tregs, thereby increasing the proportion of Teffs, but also allow IL-2 to stimulate Teff cells, thereby increasing the killing ability of Teff cells and correcting the tumor microenvironment. Therefore, the antibody has important clinical significance.

### BRIEF DESCRIPTION OF THE DRAWINGS

Embodiments of the present invention are described in detail below with reference to the attached figures, in which:
Figure 1 shows the inhibitory activity of the chimeric antibodies on the binding of IL2 to human CD25.
Figure 2 shows the binding activity of the chimeric antibodies to CHO-K1/hCD25.
Figure 3 shows the in vitro killing activity of the chimeric antibodies on Tregs.
Figure 4 shows the in vitro inhibitory activity of the chimeric antibodies on STATS phosphorylation in Treg cells stimulated by IL2.
Figure 5 shows the in vitro inhibitory activity of the humanized antibodies on STATS phosphorylation in Treg cells stimulated by IL2.
Figure 6 shows the in vitro killing activity of the humanized antibodies on Tregs.
Figure 7 shows the pharmacokinetics profiles of the humanized antibodies in wild-type mice.
Figure 8 shows the efficacy of the humanized antibodies in an animal model which were humanized mice engrafted with MC38 colon cancer cells.
Figure 9 shows the efficacy of the humanized antibodies in an animal model which were humanized mice engrafted with MC38 colon cancer cells, associated with PD. The data are shown as mean ± SEM and were analyzed by One-way ANOVA with Dunnett's multiple comparison test as compared to PBS (* p < 0.05, ** p < 0.01, *** p < 0.001, **** p < 0.0001).

### DETAILED DESCRIPTION OF PREFERRED EMBODIMENTS

The present invention is illustrated below with reference to specific examples. It will be understood by those skilled in the art that these examples are merely illustrative of the present invention and do not limit the scope of the present invention in any way.

Experimental procedures in the following examples are all conventional ones, unless otherwise specified. Raw materials and reagents used in the following examples are all commercially available products, unless otherwise specified.

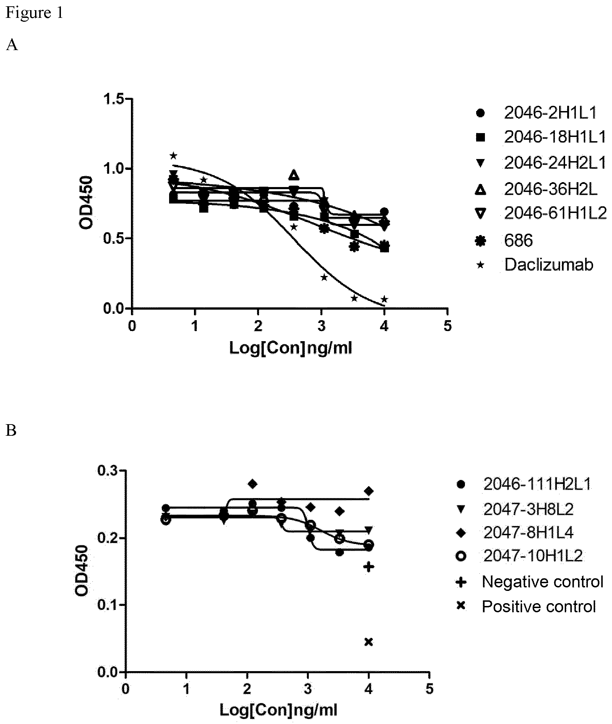

"Antibody 686" or "686" as described in the present disclosure refers to aCD25-a-686 provided in CN112020514A, and sequences of aCD25-a-686 are shown in Fig. 1 in that document.

### Example 1: Murine antibody screening and identification

### 1.1: Animal immunization

CHO-K1 cells were used to express human CD25 (NP_000408.1) (Met 1-Cys 213) C-terminally fused with 6-histidine tag. 4 BALB/c mice were immunized according to a conventional immunization program with Freund's adjuvant. The immunization was performed in two batches of mice including 2 mice in each, with two weeks between the batches. Each batch of mice was immunized 4 times, tested by ELISA using the human CD25-his, and terminally immunized if any mouse had a serum titer >1:1000; and 3-4 days later spleens were collected.

### 1.2: B cell panning and culture

Feeder cells were plated using culture medium into 4 10-cm dishes (Corning, cat. 430167) two days before formal experiment was performed. One day before the formal experiment, the feeder cells were plated into 40 96-well plates (Corning, cat. 3599) at 10000 cells/well and 100 µL/well. In the meantime, B cells harvested from those immunized mice were panned with coated antigen human CD25-his, and then the obtained B cells were plated into the 96-well plates coated with feeder cells above. The cells were cultured at 37 °C 5% CO₂ for approximately 10-14 days, and B cell culture supernatants in the wells were collected for screening via ELISA and a cell-based assay.

### 1.3: Screening of supernatants containing murine antibodies

ELISA screening of murine antibodies binding to human CD25-his:

The immunogen human CD25-his as above was diluted with a coating buffer to 1 µg/mL, added into ELISA plates at 50 µL/well and coated overnight at 4 °C. Next day, the coated plates were taken, washed with PBST for 3 times, and then incubated using a blocking buffer at room temperature for 1 h. Subsequently, the plates were washed with PBST for 3 times again; and B cell culture supernatants in the wells in which evident clones formed were added into the ELISA plates and incubated at room temperature for 1 h. The plates were washed with PBST for 3 times, and then a goat anti-mouse secondary antibody (1:10000) was added at 50 µL/well into the plates which subsequently were incubated at room temperature for 1 h. The plates were washed with PBST for 3 times, and TMB was added at 50 µL/well to develop color in dark for 10 min. Afterwards, 2 M sulfuric acid was added at 100 µL/well to stop reaction; and OD values at 450 nm were read by a microplate reader. An OD value 10 times greater than that of negative control was used as a judgment standard for positivity, and positive clones were picked for sequencing of the antibodies in the B cells.

### Example 2: Sequencing of the antibodies in the B cells

mRNAs were extracted from the B cell clones using PureLink^{™} RNA Mini Kit according to the instructions therein, subpackaged and stored at -80 °C. The extracted mRNAs were used as templates and reverse transcribed into cDNAs using PrimeScript^{™} II 1st Strand cDNA Synthesis Kit according to the instructions therein, which were then subpackaged and stored at -80 °C.

VH and VL sequences were amplified using Ex Taq enzyme and suitable primers with the above cDNAs as templates; and then ligated into pMD18T vector for sequencing. Upon sequencing, the VH and VL sequences were constructed to the N-terminus of hIgG 1 (SEQ ID NO: 104) and hKappa (SEQ ID NO: 105) constant regions, respectively.

### Example 3: Recombinant expression and activity detection of chimeric antibodies

### 3.1: Recombinant expression of chimeric antibodies

Light and heavy chains derived from the same clone were co-transfected in pairs into CHO-K1 cells. 24 h after the transfection, 10 µg/mL MSX was added for pressure screening. The cells, after cell density and viability recovered, were inoculated for Feed-batch expression, and supernatants when the expression was completed were centrifuged and purified by protein A. Antibodies obtained were used for quantitative screening after antibody concentrations were determined by BCA method.

Chimeric antibodies were named after the number of the cell clone from which their light and heavy chains (H+L) were derived. For example, chimeric antibody "2046-2H1L1" represents a chimeric antibody derived from the cell clone numbered 2046-2, the heavy chain of which is 2046-2H1, and the light chain of which is 2046-2L1.

### 3.2: Detection of the binding activity of chimeric antibodies to human CD25

Human CD25-his was diluted with a coating buffer to 1 µg/mL, added into 96-well plates at 50 µL/well, and coated overnight at 4 °C. Next day, the coated plates were taken, washed with PBST for 3 times, and then incubated using a blocking buffer at room temperature for 1 h. Subsequently, the plates were washed with PBST for 3 times again. Starting at 100 ng/mL, each chimeric antibody was diluted 3-fold to obtain serial dilutions of 8 concentrations in total which were then added into the 96-well plates at 50 µL/well. The plates were incubated at room temperature for 1 h, washed with PBST for 3 times; and then a goat anti-human secondary antibody (1:10000) was added at 50 µL/well into the 96-well plates, which subsequently were incubated at room temperature for 1 h. Then the plates were washed with PBST for 3 times, and TMB was added at 50 µL/well into the 96-well plates to develop color in dark for 10 min. Afterwards, 2 M sulfuric acid was added at 100 µL/well to stop reaction; and OD values at 450 nm were read by a microplate reader. Results are shown in Table 1. As can be seen from Table 1, the obtained chimeric antibodies have a binding activity to human CD25 which is comparable to that of 686.

**Table 1: Binding activity of chimeric antibodies to human CD25 and relative activity of the antibodies compared with 686**

| Antibody | EC₅₀ (ng/mL) | Relative activity (%) |
|---|---|---|
| 2046-2H1L1 | 105.1 | 98.95% |
| 2046-36H2L1 | 102.9 | 101.07% |
| 2046-61H1L2 | 144 | 72.22% |
| 2046-18H1L1 | 102 | 101.96% |
| 2046-24H2L1 | 105.9 | 98.21% |
| 2046-111H2L1 | 98.87 | 105.19% |
| 2047-3H8L2 | 111.1 | 93.61% |
| 2047-10H1L2 | 185.8 | 55.97% |

### 3.3: Detection of the inhibitory activity of chimeric antibodies on the binding of IL2 to human CD25

Human CD25-his protein was diluted with a coating buffer to 1 µg/mL, added into 96-well plates at 50 µL/well and coated overnight in a refrigerator at 4 °C. The coated plates were washed with PBST for 3 times, and then were incubated at room temperature for 1 h after a blocking buffer was added into the plates at 100 µL/well; and subsequently the plates were washed with PBST for 3 times again. Biotin-IL2 (prepared by the method as follows: Sulfo-NHS-LC-LC-Biotin and IL2 molecule were mixed uniformly at a molar ratio of 10:1 and then reacted at room temperature for 30 minutes; the obtained labeled solution was subjected to ultrafiltration in PBS, to remove redundant labeling agent, and then were subpackaged and cryopreserved in ultra low temperature refrigerator) was diluted to 150 ng/mL in a diluting solution (PBS+0.5% BSA+ 0.05% Tween 20); and each chimeric antibody was diluted in the diluting solution to a starting concentration of 10 µg/mL, and then was diluted 3-fold to obtain serial dilutions of 8 concentrations in total. The diluted Biotin-IL2 and each diluted chimeric antibody were mixed in a 1:1 ratio, and then the mixtures were added into the 96-well plates at 50 µL/well, which were subsequently incubated at room temperature for 1 h. The plates were washed with PBST for 3 times, and then HRP-conjugated streptavidin (1:10000) was added at 50 µL/well and the plates were incubated at room temperature for 1 h. The 96-well plates were washed with PBST for 3 times again, and TMB was added at 50 µL/well to develop color in dark for 10 min. Afterwards, 2 M sulfuric acid was added at 100 µL/well to stop reaction; and OD values at 450 nm were read by a microplate reader. Results are shown in Figure 1. As can be seen from Figure 1, like 686 (CN112020514A), the obtained chimeric antibodies are all non-IL2 blocking antibodies.

### 3.4: Detection of the binding activity of chimeric antibodies to CHO-K1/hCD25

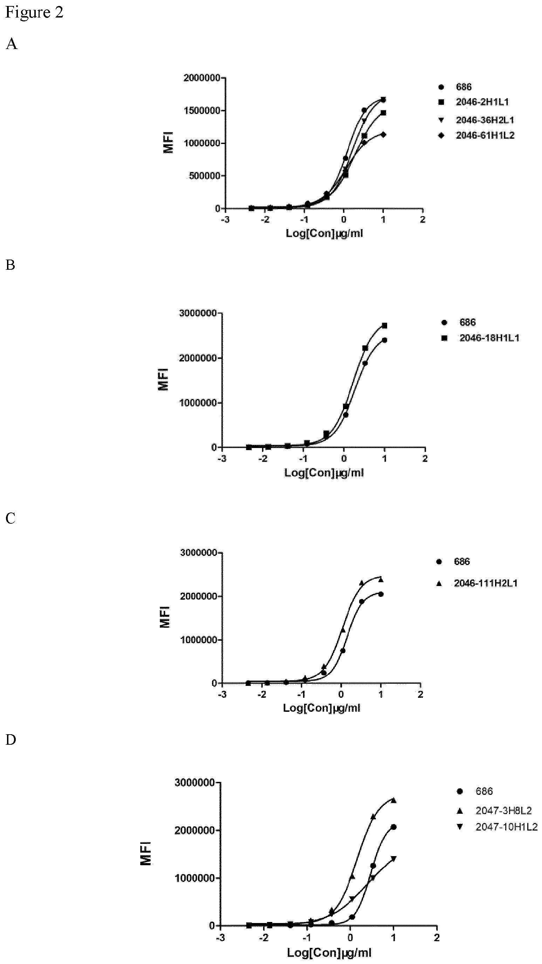

An appropriate amount of CHO-K1/hCD25 cells were collected, centrifuged at 1500 rpm for 5 min, and washed with PBS for 2 times. The cells then were resuspended in PBS at a density of 2.5 × 10⁶ cells/ml, and 100 µl was added to each well of 96-well V-shaped bottom plates. Starting at 20 µg/mL, each chimeric antibody was diluted 3-fold to obtain serial dilutions of 8 concentrations in total which were then added into the cells in the 96-well plates at 100 µL/well. The antibodies and the cells were mixed well and then the obtained mixtures comprise the antibodies in final concentrations of 10 µg/mL, 3.33 µg/mL, 1.11 µg/mL, 0.37 µg/mL, 0.12 µg/mL, 0.041 µg/mL, 0.014 µg/mL, and 0.0046 µg/mL, respectively. A well containing no primary antibody was set up as negative control. After incubation for 60 min at 4 °C, the incubated cell suspensions were centrifuged and washed with PBS for 2 times, 200 µl/well, and then the cells were resuspended in 200 µl/well PBS. FITC labeled donkey anti-human secondary antibody (Jackson, cat. 709-095- 098) was added at 5 µg/ml into the cells, mixed well and incubated for 60 min at 4 °C. Then the incubated cell suspensions were centrifuged, washed with PBS for 2 times, and the cells were resuspended in 200 µl/well PBS again. Detection was performed using a flow cytometer and the results are shown in Table 2 and Figure 2. As can be seen from Table 2 and Figure 2, in the 8 chimeric antibodies, antibodies 2046-24H2L1, 2046-111H2L1, 2047-3H8L2 and 2047-10H1L2 have a binding activity to CHO-K1/hCD25 which was obviously higher than that of 686, indicating that those four chimeric antibodies have better hCD25 binding activity than 686.

**Table 2: Binding activity of chimeric antibodies to CHO-K1/hCD25 and relative activity of the antibodies compared with 686**

| Antibody | EC₅₀ (ng/mL) | Relative activity (%) |
|---|---|---|
| 2046-2H1L1 | 1.923 | 63.81% |
| 2046-3 6H2L1 | 1.673 | 73.34% |
| 2046-61H1L2 | 1.145 | 107.16% |
| 2046-18H1L1 | 1.706 | 109.91% |
| 2046-24H2L1 | 1.427 | 131.39% |
| 2046-111H2L1 | 1.086 | 132.04% |
| 2047-3H8L2 | 1.434 | 205.23% |
| 2047-10H1L2 | 2.455 | 119.88% |

### 3.5: Detection of the affinity of chimeric antibodies

Experiments for measuring interactions between the chimeric antibodies and human CD25-his were performed using BIAcore instrument X100. All the experiments were operated in HBS-EP (1×) buffer at 25 °C. An anti-histidine antibody was immobilized to the surface of CM5 chips by amino coupling method, and then human CD25-his at a concentration of 1 µg/ml was loaded and captured for 60 s. Starting at 50 nM, each solution containing an anti-CD25 antibody was 2-fold diluted to obtain serial dilutions of 6 concentrations in total. The dilutions were injected, and association was monitored for 180 s, and dissociation was monitored for 600 s. The chips were regenerated by injecting a glycine solution at pH 1.5. Kinetic data were analyzed using a 1:1 binding model. Specific results are shown in Table 3. As can be seen from Table 3, chimeric antibodies 2046-18H1L1, 2046-111H2L1, 2047-3H8L2, and the like have obviously higher affinity than 686.

**Table 3: Affinity of the chimeric antibodies detected**

| Antibody | ka (1/Ms) | kd (1/s) | KD (M) |
|---|---|---|---|
| 2046-2H1L1 | 7.45E+05 | 0.003179 | 4.27E-09 |
| 2046-3 6H2L1 | 3.80E+06 | 0.00933 | 2.45E-09 |
| 2046-61H1L2 | 1.28E+06 | 0.0121 | 9.44E-09 |
| 2046-18H1L1 | 4.63E+05 | 3.26E-04 | 7.03E-10 |
| 2046-24H2L1 | 4.58E+05 | 0.003493 | 7.63E-09 |
| 2046-111H2L1 | 5.26E+05 | 4.31E-04 | 8.19E-10 |
| 2047-3H8L2 | 2.36E+05 | 7.26E-05 | 3.08E-10 |
| 2047-10H1L2 | 7.88E+05 | 1.22E-02 | 1.55E-08 |
| 686 | 1.48E+06 | 0.001667 | 1.13E-09 |

### 3.6: Activity of chimeric antibodies in killing Treg cells in vitro

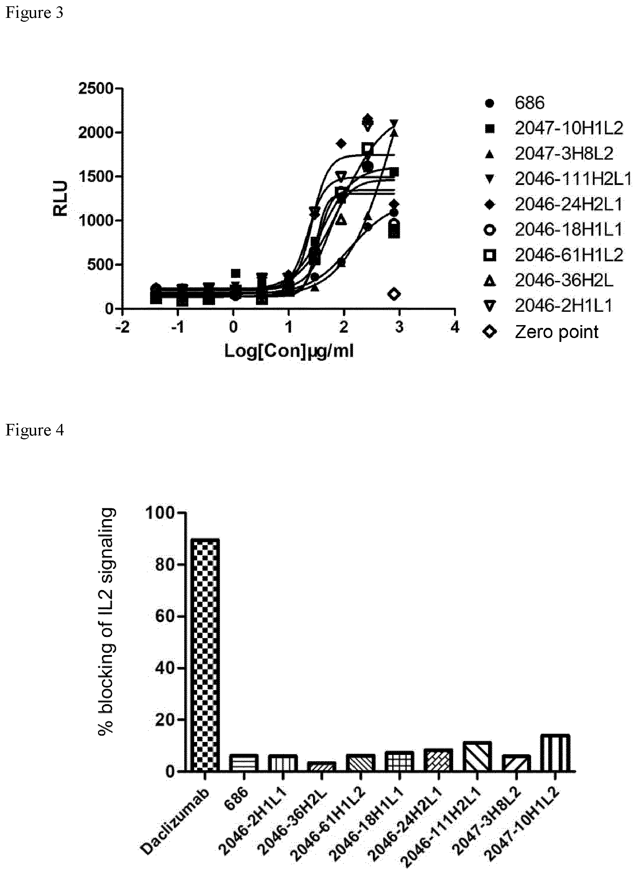

CD4⁺CD25⁺Treg cells were isolated from human PBMCs. Chimeric antibodies were diluted in 96-well plates and added into 384-well white/clear bottom cell culture plates (Corning, cat. 3765) at 20 µl/well, which were then placed at 37 °C, 5% CO₂. ADCC effector cells (jurkat/CD16a/NFAT-luc cells) were counted, and an appropriate amount of the cells were centrifuged at 1500 rpm for 5 min, and then were resuspended in a culture medium to obtain a density of 2.4×10⁶ cells/ml. A suspension of Treg cells at a density of 4×10⁵ cells/ml was mixed with the suspension of ADCC effector cells at a density of 2.4×10⁶ cells/ml in a 1:1 volume ratio, so that in the obtained suspension, the density of Treg cells was 2×10⁵ cells/ml and the density of ADCC effector cells was 1.2×10⁶ cells/ml. The cells (E:T = 6:1) were added to the 384-well white/clear cell culture plates at 20 µl/well, to which the antibodies had been added, and incubated overnight (22-24 h) at 37 °C, 5% CO₂. After the incubation, Bio-Lite Luciferase Assay System was added into the plates at 40 µl/well, and chemiluminescence values were detected in a microplate reader. Specific results are shown in Table 4 and Figure 3. As can be seen from Table 4 and Figure 3, chimeric antibodies 2046-2H1L1, 2046-36H2L1, 2046-61H1L2, 2046-18H1L1, 2046-24H2L1, 2046-111H2L1, 2047-10H1L2 and the like have obviously better activity than 686 in killing Tregs in vitro.

**Table 4: Activity of chimeric antibodies in killing Tregs in vitro and relative activity of the antibodies compared with 686**

| Antibody | EC₅₀ (µg/mL) | Relative activity (%) |
|---|---|---|
| 686 | 129.9 | 100.00% |
| 2046-2H1L1 | 22.25 | 583.82% |
| 2046-3 6H2L1 | 52.39 | 247.95% |
| 2046-61H1L2 | 34.14 | 380.49% |
| 2046-18H1L1 | 31.69 | 409.91% |
| 2046-24H2L1 | 26.47 | 490.74% |
| 2046-111H2L1 | 89.8 | 144.65% |
| 2047-3H8L2 | 856.6 | 15.16% |
| 2047-10H1L2 | 41.43 | 313.54% |

### 3.7: Activity of chimeric antibodies in inhibiting STATS phosphorylation in Treg cells stimulated by IL2 in vitro

CD4⁺CD25⁺Treg cells were isolated from human PBMCs, and cultured in 1.5 ml EP tubes. Anti-CD25 antibodies (Daclizumab was used as a negative control, and 686 was used as a positive control) were diluted with RPMI-1640+10% FBS to obtain a concentration of 10 µg/ml. The antibodies were added into the EP tubes at 200 µl/tube and incubated for 30 min. Then the supernatants in the tubes were removed by centrifugation, and 200 µl of IL-2 at a concentration of 10 ng/ml was added into each tube which was then incubated for 15 min at 37 °C. After the incubation, the tubes were placed on an ice bath prepared in advance to stop the reaction; at the meantime, a centrifuge was set to 4 °C for precooling. Then the CD4⁺CD25⁺Treg cells in the tubes were centrifuged at 300×g, at 4 °C for 5 min, and the supernatants therein were discarded. After that, the cells were washed with PBS+3% FBS precooled on ice once, and fixed with 1.5% paraformaldehyde (precooled at 4 °C which was added into the cells at 100 µl/tube) for 20 min. Afterwards, the cells were washed with 900 µl of PBS+3% FBS added and were then centrifuged and supernatants were discarded. Next, ice-cold methanol precooled at -20 °C overnight was added at 100 µl/tube into the tubes, which were then placed on ice for performing permeabilization for 5 min. Then the fixed and permeabilized CD4⁺CD25⁺Treg cells were washed with 900 µl of PBS+3% FBS added and were then centrifuged at 300×g for 5 min, and supernatants were discarded. The cells were washed once with 500 µl of PBS+3% FBS added again, and then were centrifuged at 300×g for 5 min. After that, the cells were resuspended in 100 µl of PBS, and Alexa Fluor647 Mouse Anti-Stat5 (pY694) antibody was added into the cells, at 5 µl/tube. The cells and the antibody were mixed well, and incubated for 30 min - 1 h at room temperature in dark. The supernatants were removed by centrifugation, and the cells were washed 2 times with PBS and resuspended in 200 µl of PBS. Then on-machine detection was performed. Results are shown in Figure 4. As can be seen from Figure 4, like 686, the obtained chimeric antibodies are all non-IL2 blocking antibodies.

### 3.8: Cross reactivity in vitro of chimeric antibodies towards mouse and cyno CD25

Mouse CD25-his (UniProtKB-P01590, Met1-Lys213) and cyno CD25-his (NCBI-H6WS54, Met1-Arg213) were diluted with a coating buffer to 1 µg/mL, added into 96-well plates at 50 µL/well and coated overnight at 4 °C. Next day, the coated plates were taken, washed with PBST for 3 times, and then incubated with a blocking buffer for 1 h at room temperature and then were washed with PBST for 3 times again. Starting at 100 ng/mL, each chimeric antibody was diluted 3-fold to obtain serial dilutions of 8 concentrations in total which were then added into the 96-well plates at 50 µL/well. The plates were incubated at room temperature for 1 h, washed with PBST for 3 times; and then a goat anti-human secondary antibody (1:10000) was added at 50 µL/well into the 96-well plates, which subsequently were incubated at room temperature for 1 h. Then the 96-well plates were washed with PBST for 3 times, and TMB was added at 50 µL/well to develop color in dark for 10 min. Afterwards, 2 M sulfuric acid was added at 100 µL/well to stop reaction; and OD values at 450 nm were read by a microplate reader. Results are shown in Table 5. As can be seen from Table 5, the obtained chimeric antibodies have cross reactivity towards cyno CD25, but no cross reactivity towards mouse CD25.

**Table 5: Cross reactivity in vitro of chimeric antibodies towards mouse and cyno CD25**

| **Antibody** | **Binding to CD25 from different species** | |
|---|---|---|
| | **Binding to mouse CD25-his** | **Binding to cyno CD25-his EC₅₀ (µg/ml)** |
| 2046-2H1L1 | N/A | 0.0594 |
| 2046-3 6H2L1 | N/A | 0.0549 |
| 2046-61H1L2 | N/A | 0.0601 |
| 2046-18H1L1 | N/A | 0.0483 |
| 2046-24H2L1 | N/A | 0.0424 |
| 2046-111H2L1 | N/A | 0.0447 |
| 2047-3H8L2 | N/A | 0.1890 |
| 2047-10H1L2 | N/A | 2.6020 |

### Example 4: Humanization of chimeric antibodies

8 chimeric antibodies 2046-2H1L1, 2046-36H2L1, 2046-61H1L2, 2046-18H1L1, 2046-24H2L1, 2046-111H2L1, 2047-3H8L2, and 2047-10H1L2 were selected for humanization design.

Heavy and light chain variable region sequences of the 8 chimeric antibodies were compared to human germline sequences by blast searches in IMGT database. Redundant genes as well as those with unpaired cysteines were removed from the human germline genes. The human germline gene in the remaining ones having the most matched framework and CDR regions was selected and their framework regions were used as human acceptor frameworks. FR-4 was selected based on sequence similarity of IGHJ/IGJK germline genes. Humanized sequences obtained for the 8 chimeric antibodies 2046-2H1L1, 2046-36H2L1, 2046-61H1L2, 2046-18H1L1, 2046-24H2L1, 2046-111H2L1, 2047-3H8L2, and 2047-10H1L2 are shown in Tables 6 to 13, respectively, in which the HZ0 versions represent only CDR-grafted ones, the HZ1 versions have back mutation(s) introduced, and the HZ2 and further versions have mutation(s) which are attempted at PTM sites present in the sequences; and respective CDRs (defined by enhanced Chothia/AbM) are underlined.

**Table 6: Humanized sequences obtained for heavy and light variable regions of 2046-2H1L1**

| VH | Sequence | VL | Sequence |
|---|---|---|---|
| 2046-2H1 (SEQ ID NO. 1) | DVQLQESGPGLVKPSQSLSLTCT VTGYSITSDYAWNWIRHFPGNKL EWMGYITYSGSTVYNPSLKSRISI TRDTSKNHFFLQLNSVTTEDTAT YYCARWLLQIYYAMDYWGQGT SVTVSS CDRs: SEQ ID NO. 63/64/65 | 2046-2L1 (SEQ NO. 26) | DVVMTQTPLSLPVSLGDQASISC RSSQSLVHSNGNTYLHWYLQKP GQSPKLLIYKVSNRFSGVPDRFS GSGSGTDFTLKISRVEAEDLGVY FCSQSTHVPLTFGAGTKLELK CDRs: SEQ ID NO. 82/83/84 |
| IGHV4-30-4* 01 /IGHJ4*01 | QVQLQESGPGLVKPSQTLSLTCT VSGGSISSGDYYWSWIRQPPGKG LEWIGYIYYSGSTYYNPSLKSRV TISVDTSKNQFSLKLSSVTAADTA VYYCARYFDYWGQGTLVTVSS | IGKV2-30*0 2/IGKJ2*01 | DVVMTQSPLSLPVTLGQPASISC RSSQSLVHSDGNTYLNWFQQRP GQSPRRLIYKVSNRDSGVPDRFS GSGSGTDFTLKISRVEAEDVGVY YCMQGTHWPYTFGQGTKLEIK |
| 2046-2H1-H Z0 (SEQ ID NO. 2) | EVQLQESGPGLVKPSQTLSLTCT VSGYSITSDYAWNWIRQPPGKGL EWIGYITYSGSTVYNPSLKSRVTI SVDTSKNQFSLKLSSVTAADTAV YYCARWLLQIYYAMDYWGQGT LVTVSS | 2046-2L1-H Z0 (SEQ NO. 27) | DVVMTQSPLSLPVTLGQPASISC RSSQSLVHSNGNTYLHWFQQRP GQSPRRLIYKVSNRFSGVPDRFS GSGSGTDFTLKISRVEAEDVGVY YCSQSTHVPLTFGQGTKLEIK CDRs: SEQ ID NO. 82/83/84 |
| | CDRs: SEQ ID NO. 63/64/65 | | |
| 2046-2H1-H Z1 (SEQ ID NO. 3) | EVQLQESGPGLVKPSQTLSLTCT VSGYSITSDYAWNWIRQPPGKGL EWIGYITYSGSTVYNPSLKSRVTI SRDTSKNQFSLKLSSVTAADTAV YYCARWLLQIYYAMDYWGQGT LVTVSS | 2046-2L1-H Z1 (SEQ NO. 28) | DVVMTQSPLSLPVTLGQPASISC RSSQSLVHSNGNTYLHWYQQRP GQSPRLLIYKVSNRFSGVPDRFS GSGSGTDFTLKISRVEAEDLGVY FCSQSTHVPLTFGQGTKLEIK CDRs: SEQ ID NO. 82/83/84 |
| | CDRs: SEQ ID NO. 63/64/65 | 2046-2L1-H Z2 (SEQ NO. 29) | DVVMTQSPLSLPVTLGQPASISC RSSQSLVHSSGNTYLHWYQQRP GQSPRLLIYKVSNRFSGVPDRFS GSGSGTDFTLKISRVEAEDLGVY FCSQSTHVPLTFGQGTKLEIK CDRs: SEQ ID NO. 85/83/84 |

**Table 7: Humanized sequences obtained for heavy and light variable regions of 2046-18H1L1**

| VH | Sequence | VL | Sequence |
|---|---|---|---|
| 2046-18H1 (SEQ ID NO. 4) | DVQLQESGPGLVKPSQSLSLTCT VTGYSITSDYAWNWIRQFPGNKL EWMGYISYSGITIYNPSLKSRISIT RDTSKNQFFLQLNSVTTEDTATY YCARWLLQIYYAMDYWGQGTS VTVSS CDRs: SEQ ID NO. 63/66/65 | 2046-18L1 (SEQ ID NO. 30) | DVVMTQTPLSLPVSLGDQASIS CSSSQSLVHSNGNTYLHWYLH KPGQSPKLLIYKVSNRFSGVPD RFSGSGSGTDFTLKISRVEAED LGVYFCSQSTHVPLTFGAGTKL ELK CDRs: SEQ ID NO. 86/83/84 |
| IGHV4-30-4* 01/IGHJ4*01 | QVQLQESGPGLVKPSQTLSLTCT VSGGSISSGDYYWSWIRQPPGKG LEWIGYIYYSGSTYYNPSLKSRV TISVDTSKNQFSLKLSSVTAADTA VYYCARYFDYWGQGTLVTVSS | IGKV2-30*02 /IGKJ2*01 | DVVMTQSPLSLPVTLGQPASIS CRSSQSLVHSDGNTYLNWFQQ RPGQSPRRLIYKVSNRDSGVPD RFSGSGSGTDFTLKISRVEAED VGVYYCMQGTHWPYTFGQGT KLEIK |
| 2046-18H 1-HZ0 (SEQ ID NO. | | 2046-18L1-H Z0 (SEQ ID NO. | DVVMTQSPLSLPVTLGQPASIS CSSSQSLVHSNGNTYLHWFQQ RPGQSPRRLIYKVSNRFSGVPD |
| 5) | VDTSKNQFSLKLSSVTAADTAVY YCARWLLQIYYAMDYWGQGTL VTVSS CDRs: SEQ ID NO. 63/66/65 | 31) | RFSGSGSGTDFTLKISRVEAED VGVYYCSQSTHVPLTFGQGTK LEIK CDRs: SEQ ID NO. 86/83/84 |
| 2046-18H1-H Z1 (SEQ ID NO. 6) | EVQLQESGPGLVKPSQTLSLTCT VSGYSITSDYAWNWIRQPPGKGL EWIGYISYSGITIYNPSLKSRVTIS RDTSKNQFSLKLSSVTAADTAVY YCARWLLQIYYAMDYWGQGTL VTVSS CDRs: SEQ ID NO. 63/66/65 | 2046-18L1-H Z1 (SEQ ID NO. 32) | DVVMTQSPLSLPVTLGQPASIS CSSSQSLVHSNGNTYLHWYQQ RPGQSPRLLIYKVSNRFSGVPD RFSGSGSGTDFTLKISRVEAEDL GVYFCSQSTHVPLTFGQGTKLE IK CDRs: SEQ ID NO. 86/83/84 |
| | | 2046-18L1-H Z2 (SEQ ID NO. 33) | DVVMTQSPLSLPVTLGQPASIS CSSSQSLVHSSGNTYLHWYQQ RPGQSPRLLIYKVSNRFSGVPD RFSGSGSGTDFTLKISRVEAEDL GVYFCSQSTHVPLTFGQGTKLE IK CDRs: SEQ ID NO. 87/83/84 |
| | | 2046-18L1-T2 (SEQ ID NO 34) | EIVLTQSPGTLSLSPGERATLSC SSSQSLVHSNGNTYLHWYQQK PGQAPRLLIYKVSNRFSGIPDRF SGSGSGTDFTLTISRLEPEDFAV YYCSQSTHVPLTFGQGTKLEIK CDRs: SEQ ID NO. 86/83/84 |

**Table 8: Humanized sequences obtained for heavy and light variable regions of 2046-24H2L1**

| VH | Sequence | VL | Sequence |
|---|---|---|---|
| 2046-24H2 (SEQ ID NO. 7) | DVQLQESGPGLVKPSQSLSLTCT VTGYSITSDYAWNWIRQFPGNKL EWMGYISYGGTTIYNPSLKSRISI TRDTSKNQFFLQLNSVTTEDTAT YYCARWLLQNYYAMDYWGQG | 2046-24L1 (SEQ ID NO. 35) | DVVMTQTPLSLPVSLGDQASIS CRSSQSLVHSNGNTYLHWYLQ KPGQSPKVLIYKVSNRFSGVPD RFSGSGSGTDFTLKISRVEAED LGVYFCSQNTHVPLTFGAGTK |
| | TSVTVSS CDRs: SEQ ID NO. 63/67/68 | | LELK CDRs: SEQ ID NO. 82/83/88 |
| IGHV4-30-4* 01/IGHJ4*01 | QVQLQESGPGLVKPSQTLSLTCT [?] VSGGSISSGDYYWSWIRQPPGKG [?] LEWIGYIYYSGSTYYNPSLKSRV TISVDTSKNQFSLKLSSVTAADTA VYYCARYFDYWGQGTLVTVSS | IGKV2-30*02 /IGKJ2*01 | DVVMTQSPLSLPVTLGQPASIS CRSSQSLVHSDGNTYLNWFQQ RPGQSPRRLIYKVSNRDSGVPD RFSGSGSGTDFTLKISRVEAED VGVYYCMQGTHWPYTFGQGT KLEIK |
| 2046-24H2-H Z0 (SEQ ID NO. 8) | EVQLQESGPGLVKPSQTLSLTCT 2 VSGYSITSDYAWNWIRQPPGKGL 2 EWIGYISYGGTTIYNPSLKSRVTIS[?] VDTSKNQFSLKLSSVTAADTAVY 3 YCARWLLQNYYAMDYWGQGTL VTVSS CDRs: SEQ ID NO. 63/67/68 | 2046-24L1-H Z0 (SEQ ID NO. 56) | DVVMTQSPLSLPVTLGQPASIS CRSSQSLVHSNGNTYLHWFQQ RPGQSPRRLIYKVSNRFSGVPD RFSGSGSGTDFTLKISRVEAED VGVYYCSQNTHVPLTFGQGTK LEIK CDRs: SEQ ID NO. 82/83/88 |
| 2046-24H2-H Z1 (SEQ ID NO. 9) | EVQLQESGPGLVKPSQTLSLTCT VSGYSITSDYAWNWIRQPPGKGL EWIGYISYGGTTIYNPSLKSRVTIS RDTSKNQFSLKLSSVTAADTAVY YCARWLLQNYYAMDYWGQGTL VTVSS CDRs: SEQ ID NO. 63/67/68 | 2046-24L1-H Z1 (SEQ ID NO. 57) | DVVMTQSPLSLPVTLGQPASIS CRSSQSLVHSNGNTYLHWYQQ RPGQSPRVLIYKVSNRFSGVPD RFSGSGSGTDFTLKISRVEAEDL GVYFCSQNTHVPLTFGQGTKL EIK CDRs: SEQ ID NO. 82/83/88 |
| | | 2046-24L1-H Z2 (SEQ ID NO. 38) | DVVMTQSPLSLPVTLGQPASIS CRSSQSLVHSSGNTYLHWYQQ RPGQSPRVLIYKVSNRFSGVPD RFSGSGSGTDFTLKISRVEAEDL GVYFCSQNTHVPLTFGQGTKL EIK CDRs: SEQ ID NO. 85/83/88 |

**Table 9: Humanized sequences obtained for heavy and light variable regions of 2046-36H2L1**

| VH | Sequence | VL | Sequence |
|---|---|---|---|
| 2046-36H2 (SEQ ID NO. 10) | QIQLVQSGPELKKPGETVRISCKA[?] SGYTFTTAGMQWVQKMPGKGL ( KWIGWINTHSGVPKYAEDFKGR 3 FAFSLETSASTAYLQISNLKNEDT ATYFCASDYAFGYWGQGTLVTV SA CDRs: SEQ ID NO. 69/70/71 | 2046-3 6L1 (SEQ ID NO. 19) | DVVMTQTPLTLSVTIGQPASIS CKSSQSLLYSNGKTYLYWLFQ RPGQSPKRLIYLVSKLDSGVPD RFTGSGSGTDFTLKISRVEAED LGVYYCVQGTHFPYTFGGGTK LEIK CDRs: SEQ ID NO. 89/90/91 |
| IGHV7-4-1*0 2/IGHJ4*01 | QVQLVQSGSELKKPGASVKVSC [?] KASGYTFTSYAMNWVRQAPGQG[?] LEWMGWINTNTGNPTYAQGFTG RFVFSLDTSVSTAYLQISSLKAED TAVYYCARYFDYWGQGTLVTVS S | IGKV2-30*02 /IGKJ2*01 | DVVMTQSPLSLPVTLGQPASIS CRSSQSLVHSDGNTYLNWFQQ RPGQSPRRLIYKVSNRDSGVPD RFSGSGSGTDFTLKISRVEAED VGVYYCMQGTHWPYTFGQGT KLEIK I |
| 2046-36H2-H Z0 (SEQ ID NO. 11) | EVQLVQSGSELKKPGASVKVSCK 2[?] ASGYTFTTAGMQWVRQAPGQGL [?] EWMGWINTHSGVPKYAEDFKGR [?] FVFSLDTSVSTAYLQISSLKAEDT 4[?] AVYYCARDYAFGYWGQGTLVTV SS CDRs: SEQ ID NO. 69/70/71 | 2046-36L-HZ 0 (SEQ ID NO. 40) | DVVMTQSPLSLPVTLGQPASIS CKSSQSLLYSNGKTYLYWFQQ RPGQSPRRLIYLVSKLDSGVPD RFSGSGSGTDFTLKISRVEAED VGVYYCVQGTHFPYTFGQGTK LEIK CDRs: SEQ ID NO. 89/90/91 |
| 2046-36H2-H Z1 (SEQ ID NO. 12) | EIQLVQSGSELKKPGASVKVSCK 2 ASGYTFTTAGMQWVRQAPGQGL [?] EWMGWINTHSGVPKYAEDFKGR [?] FVFSLDTSVSTAYLQISSLKAEDT 4 AVYYCASDYAFGYWGQGTLVTV SS CDRs: SEQ ID NO. 69/70/71 | 2046-36L-HZ 1 (SEQ ID NO. 41) | DVVMTQSPLSLPVTLGQPASIS CKSSQSLLYSNGKTYLYWLQQ RPGQSPRRLIYLVSKLDSGVPD RFSGSGSGTDFTLKISRVEAEDL GVYYCVQGTHFPYTFGQGTKL EIK CDRs: SEQ ID NO. 89/90/91 |
| | | 2046-36L-HZ 2 (SEQ ID NO. 42) | DVVMTQSPLSLPVTLGQPASIS CKSSQSLLYSSGKTYLYWLQQ RPGQSPRRLIYLVSKLDSGVPD RFSGSGSGTDFTLKISRVEAEDL GVYYCVQGTHFPYTFGQGTKL |
| | | | EIK CDRs: SEQ ID NO. 92/90/91 |
| | | 2046-36L-HZ 3 (SEQ ID NO. 43) | DVVMTQSPLSLPVTLGQPASIS CKSSQSLLYSSGKTYLYWLQQ RPGQSPRRLIYLVSKLDAGVPD RFSGSGSGTDFTLKISRVEAEDL GVYYCVQGTHFPYTFGQGTKL EIK CDRs: SEQ ID NO. 92/93/91 |

**Table 10: Humanized sequences obtained for heavy and light variable regions of 2046-61H1L2**

| VH | Sequence | VL | Sequence |
|---|---|---|---|
| 2046-61H1 (SEQ ID NO. 13) | DVQLQESGPGLVKPSQSLSLTCT VTGYSITSDYAWNWIRQFPGNKL EWMGYISYSGGTIYNPSLKSRISI TRDTSKNQFFLQLNSVTTEDTAT YYCARWLLQNYYAMDYWGQG TSVTVSS CDRs: SEQ ID NO. 63/72/68 | 2046-61L2 (SEQ ID NO. 44) | DVVMTQTPLSLPVSLGDQASIS CRSSQSLVHSNGNTYLHWNLQ KPGQSPKLLIYKVSNRFSGVPD RFSGSGSGTDFTLKISRVEAED LGVYFCSQSTHVPLTFSAGTKL ELK CDRs: SEQ ID NO. 82/83/84 |
| IGHV4-30-4* 01 /IGHJ4 * 01 | QVQLQESGPGLVKPSQTLSLTCT VSGGSISSGDYYWSWIRQPPGKG LEWIGYIYYSGSTYYNPSLKSRV TISVDTSKNQFSLKLSSVTAADTA VYYCARDYWGQGTLVTVSS | IGKV2-30*02 /IGKJ2*01 | DVVMTQSPLSLPVTLGQPASIS CRSSQSLVHSDGNTYLNWFQQ RPGQSPRRLIYKVSNRDSGVPD RFSGSGSGTDFTLKISRVEAED VGVYYCMQGTHWPYTFGQGT KLEIK |
| 2046-61H1-H Z0 (SEQ ID NO. 14) | EVQLQESGPGLVKPSQTLSLTCT VSGYSITSDYAWNWIRQPPGKGL EWIGYISYSGGTIYNPSLKSRVTIS VDTSKNQFSLKLSSVTAADTAVY YCARWLLQNYYAMDYWGQGTL VTVSS CDRs: SEQ ID NO. 63/72/68 | 2046-61L2-H Z0 (SEQ ID NO. 45) | DVVMTQSPLSLPVTLGQPASIS CRSSQSLVHSNGNTYLHWFQQ RPGQSPRRLIYKVSNRFSGVPD RFSGSGSGTDFTLKISRVEAED VGVYYCSQSTHVPLTFGQGTK LEIK CDRs: SEQ ID NO. 82/83/84 |
| 2046-61H1-H Z1 (SEQ ID NO. 15) | EVQLQESGPGLVKPSQTLSLTCT VSGYSITSDYAWNWIRQPPGKGL EWIGYISYSGGTIYNPSLKSRVTIS RDTSKNQFSLKLSSVTAADTAVY YCARWLLQNYYAMDYWGQGTL VTVSS CDRs: SEQ ID NO. 63/72/68 | 2046-61L2-H Z1 (SEQ ID NO. 46) | DVVMTQSPLSLPVTLGQPASIS CRSSQSLVHSNGNTYLHWNQQ RPGQSPRLLIYKVSNRFSGVPD RFSGSGSGTDFTLKISRVEAEDL GVYFCSQSTHVPLTFGQGTKLE IK CDRs: SEQ ID NO. 82/83/84 |
| | | 2046-61L2-H Z2 (SEQ ID NO. 47) | DVVMTQSPLSLPVTLGQPASIS CRSSQSLVHSSGNTYLHWNQQ RPGQSPRLLIYKVSNRFSGVPD RFSGSGSGTDFTLKISRVEAEDL GVYFCSQSTHVPLTFGQGTKLE IK CDRs: SEQ ID NO. 85/83/84 |

**Table 11: Humanized sequences obtained for heavy and light variable regions of 2046-111H2L1**

| VH | Sequence | VL | Sequence |
|---|---|---|---|
| 2046-111H2 (SEQ ID NO. 16) | DVQLQESGPGLVKPSQSLSLTCT VTGHSITSDYAWNWIRQFPGNKL EWMGYISYSGSTIYNPSLKSRISIT RDTSKNQFFLQLNSVTTEDTATY YCARWLLQNYYAMDYWGQGTS VTVSS CDRs: SEQ ID NO. 73/74/68 | 2046-111L1 (SEQ ID NO. 48) | DVVMTQTPLSLPVSLGDQASIS CRSSQSLVHSNGNTYLHWYLR KPGQSPKLLIYKVSNRFSGVPD RFSGSGSGTDFTLKISRVEAED LGVYFCSQSTHVPLTFGAGTKL ELK CDRs: SEQ ID NO. 82/83/84 |
| IGHV4-30-4* 01 /IGHJ4 * 01 | QVQLQESGPGLVKPSQTLSLTCT VSGGSISSGDYYWSWIRQPPGKG LEWIGYIYYSGSTYYNPSLKSRV TISVDTSKNQFSLKLSSVTAADTA VYYCARDYWGQGTLVTVSS | IGKV2-30*02 /IGKJ2*01 | DVVMTQSPLSLPVTLGQPASIS CRSSQSLVHSDGNTYLNWFQQ RPGQSPRRLIYKVSNRDSGVPD RFSGSGSGTDFTLKISRVEAED VGVYYCMQGTHWPYTFGQGT KLEIK |
| 2046-111H2-HZ0 (SEQ ID NO. 17) | EVQLQESGPGLVKPSQTLSLTCT | 2046-111L1-HZ0 (SEQ ID NO. 49) | DVVMTQSPLSLPVTLGQPASIS |
| | VSGHSITSDYAWNWIRQPPGKGL EWIGYISYSGSTIYNPSLKSRVTIS VDTSKNQFSLKLSSVTAADTAVY YCARWLLQNYYAMDYWGQGTL VTVSS CDRs: SEQ ID NO. 73/74/68 | | CRSSQSLVHSNGNTYLHWFQQ RPGQSPRRLIYKVSNRFSGVPD RFSGSGSGTDFTLKISRVEAED VGVYYCSQSTHVPLTFGQGTK LEIK CDRs: SEQ ID NO. 82/83/84 |
| 2046-111H2-HZ1 (SEQ ID NO. 18) | EVQLQESGPGLVKPSQTLSLTCT VSGHSITSDYAWNWIRQPPGKGL EWIGYISYSGSTIYNPSLKSRVTIS RDTSKNQFSLKLSSVTAADTAVY YCARWLLQNYYAMDYWGQGTL VTVSS CDRs: SEQ ID NO. 73/74/68 | 2046-111L1-H Z1 (SEQ ID NO. 50) | DVVMTQSPLSLPVTLGQPASIS CRSSQSLVHSNGNTYLHWYQQ RPGQSPRLLIYKVSNRFSGVPD RFSGSGSGTDFTLKISRVEAEDL GVYFCSQSTHVPLTFGQGTKLE IK CDRs: SEQ ID NO. 82/83/84 |
| | | 2046-111L1-H Z2 (SEQ ID NO. 51) | DVVMTQSPLSLPVTLGQPASIS CRSSQSLVHSSGNTYLHWYQQ RPGQSPRLLIYKVSNRFSGVPD RFSGSGSGTDFTLKISRVEAEDL GVYFCSQSTHVPLTFGQGTKLE IK CDRs: SEQ ID NO. 85/83/84 |
| | | 2046-111L1-T 1 (SEQ ID NO. 52) | EIVLTQSPGTLSLSPGERATLSC RSSQSLVHSNGNTYLHWYQQK PGQAPRLLIYKVSNRFSGIPDRF SGSGSGTDFTLTISRLEPEDFAV YYCSQSTHVPLTFGQGTKLEIK CDRs: SEQ ID NO. 82/83/84 |

**Table 12: Humanized sequences obtained for heavy and light variable regions of 2047-3H8L2**

| VH | Sequence | VL | Sequence |
|---|---|---|---|
| 2047-3H8 (SEQ ID NO. 19) | QVQLKQSGAELVKPGASVKMSC 2[?] KTSGYTFTDYVISWVKQRTGQG [?] LEWIGEIYPGSGSTYYNEKFKGK 5[?] | 2047-3L2 (SEQ ID NO. 53) | DIVMTQSPATLSVTPGDRVSLS CRASQSISDYLHWYQQKSHES PRLLIKYASQSISGIPSRFSGSGS |
| | ATLTADISSNTAYMQLSSLTSEDS AVYFCASINFDYWGHGTTLTVSS CDRs: SEQ ID NO. 75/76/77 | | GSDFTLSINSVEPEDVGVYYCQ NGHSFPYTFGGGTKLEIK CDRs: SEQ ID NO. 94/95/96 |
| IGHV1-69-2* 01 /IGHJ4 * 01 | EVQLVQSGAEVKKPGATVKISCK [?] VSGYTFTDYYMHWVQQAPGKG LEWMGLVDPEDGETIYAEKFQGR VTITADTSTDTAYMELSSLRSEDT AVYYCATYFDYWGQGTLVTVSS | IGKV3D-7*0 1/IGKJ2*01 | EIVMTQSPATLSLSPGERATLSC RASQSVSSSYLSWYQQKPGQA PRLLIYGASTRATGIPARFSGSG SGTDFTLTISSLQPEDFAVYYCQ QDYNLPYTFGQGTKLEIK |
| 2047-3H8-H Z0 (SEQ ID NO. 20) | EVQLVQSGAEVKKPGATVKISCK [?] VSGYTFTDYVISWVQQAPGKGL [?] EWMGEIYPGSGSTYYNEKFKGR [?] VTITADTSTDTAYMELSSLRSEDT [?] AVYYCATINFDYWGQGTLVTVSS CDRs: SEQ ID NO. 75/76/77 | 2047-3L2-HZ 0 (SEQ ID NO. 54) | EIVMTQSPATLSLSPGERATLSC RASQSISDYLHWYQQKPGQAP RLLIYYASQSISGIPARFSGSGSG TDFTLTISSLQPEDFAVYYCQN GHSFPYTFGQGTKLEIK CDRs: SEQ ID NO. 94/95/96 |
| 2047-3H8-H Z1 (SEQ ID NO. 21) | EVQLVQSGAEVKKPGATVKISCK [?] TSGYTFTDYVISWVQQAPGKGLE WMGEIYPGSGSTYYNEKFKGRV [?] TITADISTNTAYMELSSLRSEDTA [?] VYYCASINFDYWGQGTLVTVSS CDRs: SEQ ID NO. 75/76/77 | 2047-3L2-HZ 1 (SEQ ID NO. 55) | EIVMTQSPATLSLSPGERATLSC RASQSISDYLHWYQQKPGQAP RLLIKYASQSISGIPARFSGSGSG SDFTLTISSLQPEDVAVYYCQN GHSFPYTFGQGTKLEIK CDRs: SEQ ID NO. 94/95/96 |
| | | 2047-3L2-HZ 2 (SEQ ID NO. 56) | EIVMTQSPATLSLSPGERATLSC RASQSISDYLHWYQQKPGQAP RLLIKYASQSISGIPARFSGSGSG SDFTLTISSLQPEDVAVYYCQSG HSFPYTFGQGTKLEIK CDRs: SEQ ID NO. 94/95/97 |
| | | 2047-3L2-HZ 3 (SEQ ID NO. 57) | EIVMTQSPATLSLSPGERATLSC RASQSISDYLHWYQQKPGQAP RLLIKYASQSISGIPARFSGSGSG SDFTLTISSLQPEDVAVYYCQN AHSFPYTFGQGTKLEIK CDRs: SEQ ID NO. 94/95/98 |

**Table 13: Humanized sequences obtained for heavy and light variable regions of 2047-10H1L2**

| VH | Sequence | VL | Sequence |
|---|---|---|---|
| 2047-10H1 (SEQ ID NO. 22) | QVQLQQSGPELVRPGVSVKISCK GSGYTFTDYAMHWVKQSHAKS LEWIGVISTYNGNTNYNQKFKG KATMTVDKSSSTAYMELARLTS EDSAIYYCARSYGNYPFDYWGQ GTTLTVSS CDRs: SEQ ID NO. 78/79/80 | 2047-10L2 (SEQ ID NO. 58) | DIKMTQSPSSMYASLGERVTIT CKASQDINSYLSWFQQKPGKS PKTLIYRANRLVDGVPSRFSGS GSGQDYSLTISSLEYEDMGIYY CLQYDEFPYTFGGGTKLEIK CDRs: SEQ ID NO. 99/100/101 |
| IGHV1-69-2* 01 /IGHJ4 * 01 | EVQLVQSGAEVKKPGATVKISCK VSGYTFTDYYMHWVQQAPGKG LEWMGLVDPEDGETIYAEKFQGR VTITADTSTDTAYMELSSLRSEDT AVYYCATYFDYWGQGTLVTVSS | IGKV1-16*01 /IGKJ2*01 | DIQMTQSPSSLSASVGDRVTITC RASQGISNYLAWFQQKPGKAP KSLIYAASSLQSGVPSRFSGSGS GTDFTLTISSLQPEDFATYYCQQ YNSYPYTFGQGTKLEIK |
| 2047-10H1-H Z0 (SEQ ID NO. 23) | EVQLVQSGAEVKKPGATVKISCK VSGYTFTDYAMHWVQQAPGKG LEWMGVISTYNGNTNYNQKFKG RVTITADTSTDTAYMELSSLRSED TAVYYCATSYGNYPFDYWGQGT LVTVSS CDRs: SEQ ID NO. 78/79/80 | 2047-10L2-H Z0 (SEQ ID NO. 59) | DIQMTQSPSSLSASVGDRVTITC KASQDINSYLSWFQQKPGKAP KSLIYRANRLVDGVPSRFSGSG SGTDFTLTISSLQPEDFATYYCL QYDEFPYTFGQGTKLEIK CDRs: SEQ ID NO. 99/100/101 |
| 2047-10H1-H Z1 (SEQ ID NO. 24) | EVQLVQSGAEVKKPGATVKISCK GSGYTFTDYAMHWVQQAPGKG LEWMGVISTYNGNTNYNQKFKG RVTITVDKSTSTAYMELSSLRSED TAVYYCARSYGNYPFDYWGQGT LVTVSS CDRs: SEQ ID NO. 78/79/80 | 2047-10L2-H Z1 (SEQ ID NO. 60) | DIQMTQSPSSLSASVGDRVTITC KASQDINSYLSWFQQKPGKAP KTLIYRANRLVDGVPSRFSGSG SGQDYTLTISSLQPEDMATYYC LQYDEFPYTFGQGTKLEIK CDRs: SEQ ID NO. 99/100/101 |
| 2047-10H1-H Z2 (SEQ ID NO. | | 2047-10L2-H Z2 (SEQ ID NO. | DIQMTQSPSSLSASVGDRVTITC KASQDITSYLSWFQQKPGKAP KTLIYRANRLVDGVPSRFSGSG |
| 25) | RVTITVDKSTSTAYMELSSLRSED TAVYYCARSYGNYPFDYWGQGT LVTVSS CDRs: SEQ ID NO. 78/81/80 | 61) | SGQDYTLTISSLQPEDMATYYC LQYDEFPYTFGQGTKLEIK CDRs: SEQ ID NO. 102/100/101 |
| | | 2047-10L2-H Z3 (SEQ ID NO. 62) | DIQMTQSPSSLSASVGDRVTITC KASQDITSYLSWFQQKPGKAP KTLIYRANRLVSGVPSRFSGSG SGQDYTLTISSLQPEDMATYYC LQYDEFPYTFGQGTKLEIK CDRs: SEQ ID NO. 102/103/101 |

### Example 5: Recombinant expression of humanized antibodies

Light and heavy chains were constructed using the heavy chain constant region as shown in SEQ ID NO: 104 and the light chain constant region as shown in SEQ ID NO: 105, and co-transfected in pairs into CHO-K1 cells. 24 h after the transfection, 10 µg/mL MSX was added for pressure screening. The cells, after cell density and viability recovered, were inoculated for Feed-batch expression, and supernatants when the expression was completed were centrifuged and purified by protein A. Antibodies obtained were used for quantitative screening after antibody concentrations were determined by BCA method.

Humanized antibodies and light and heavy chain variable regions in pairs contained by the humanized antibodies are shown in Tables 14 to 21. An antibody having a name suffixed with "ix" is a chimeric antibody accordingly.

**Table 14: Light and heavy chain variable regions in pairs of humanized antibodies derived from the cell clone numbered 2046-2**

| | | | | |
|---|---|---|---|---|
| | 2046-2L1 | 2046-2L1-HZ0 | 2046-2L1-HZ1 | 2046-2L1-HZ2 |
| 2046-2H1 | 2046-2ix | | | |
| 2046-2H1-HZ0 | | 2046-2H0L0 | 2046-2H0L1 | 2046-2H0L2 |
| 2046-2H1-HZ1 | | 2046-2H1L0 | 2046-2H1L1 | 2046-2H1L2 |

**Table 15: Light and heavy chain variable regions in pairs of humanized antibodies derived from the cell clone numbered 2046-18**

| | | | | | |
|---|---|---|---|---|---|
| | 2046-18L1 | 2046-18L1-HZ0 | 2046-18L1-HZ1 | 2046-18L1-HZ2 | 2046-18L1-T2 |
| 2046-18H1 | 2046- 18ix | | | | |
| 2046-18H1-HZ0 | | 2046-18H0L0 | 2046-18H0L1 | 2046-18H0L2 | |
| 2046-18H1-HZ1 | | 2046-18H1L0 | 2046-18H1L1 | 2046-18H1L2 | 2046-18H1L1-m2 |

**Table 16: Light and heavy chain variable regions in pairs of humanized antibodies derived from the cell clone numbered 2046-24**

| | | | | |
|---|---|---|---|---|
| | 2046-24L1 | 2046-24L1-HZ0 | 2046-24L1-HZ1 | 2046-24L1-HZ2 |
| 2046-24H2 | 2046-24ix | | | |
| 2046-24H2-HZ0 | | 2046-24H0L0 | 2046-24H0L1 | 2046-24H0L2 |
| 2046-24H2-HZ1 | | 2046-24H1L0 | 2046-24H1L1 | 2046-24H1L2 |

**Table 17: Light and heavy chain variable regions in pairs of humanized antibodies derived from the cell clone numbered 2046-36**

| | | | | | |
|---|---|---|---|---|---|
| | 2046-36L1 | 2046-36L-HZ0 | 2046-36L-HZ1 | 2046-3 6L-HZ2 | 2046-3 6L-HZ3 |
| 2046-36H2 | 2046-36ix | | | | |
| 2046-3 6H2-HZ0 | | 2046-36H0L0 | 2046-36H0L1 | 2046-36H0L2 | 2046-36H0L3 |
| 2046-3 6H2-HZ1 | | 2046-36H1L0 | 2046-36H1L1 | 2046-36H1L2 | 2046-36H1L3 |

**Table 18: Light and heavy chain variable regions in pairs of humanized antibodies derived from the cell clone numbered 2046-61**

| | | | | |
|---|---|---|---|---|
| | 2046-61L2 | 2046-61L2-HZ0 | 2046-61L2-HZ1 | 2046-61L2-HZ2 |
| 2046-61H1 | 2046-61ix | | | |
| 2046-61H1-HZ0 | | 2046-61H0L0 | 2046-61H0L1 | 2046-61H0L2 |
| 2046-61H1-HZ1 | | 2046-61H1L0 | 2046-61H1L1 | 2046-61H1L2 |

**Table 19: Light and heavy chain variable regions in pairs of humanized antibodies derived from the cell clone numbered 2046-111**

| | | | | | |
|---|---|---|---|---|---|
| | 2046-111L1 | 2046-111L1-HZ0 | 2046-111L1-HZ1 | 2046-111L1-HZ2 | 2046-111L1-T1 |
| 2046-111H2 | 2046-111ix | | | | |
| 2046-111H2-HZ0 | | 2046-111H0L0 | 2046-111H0L1 | 2046-111H0L2 | |
| 2046-111H2-HZ1 | | 2046-111H1L0 | 2046-111H1L1 | 2046-111H1L2 | 2046-111H1L1-m2 |

**Table 20: Light and heavy chain variable regions in pairs of humanized antibodies derived from the cell clone numbered 2047-3**

| | | | | | |
|---|---|---|---|---|---|
| | 2047-3L2 | 2047-3L2-HZ0 | 2047-3L2-HZ1 | 2047-3L2-HZ2 | 2047-3L2-HZ3 |
| 2047-3H8 | 2047-3ix | | | | |
| 2047-3H8-HZ0 | | 2047-3H0L0 | 2047-3H0L1 | 2047-3H0L2 | 2047-3H0L3 |
| 2047-3H8-HZ1 | | 2047-3H1L0 | 2047-3H1L1 | 2047-3H1L2 | 2047-3H1L3 |

**Table 21: Light and heavy chain variable regions in pairs of humanized antibodies derived from the cell clone numbered 2047-10**

| | | | | | |
|---|---|---|---|---|---|
| | 2047-10L2 | 2047-10L2-HZ0 | 2047-10L2-HZ1 | 2047-10L2-HZ2 | 2047-10L2-HZ3 |
| 2047-10H1 | 2047-10ix | | | | |
| 2047-10H1-HZ0 | | 2047-10H0L0 | 2047-10H0L1 | 2047-10H0L2 | 2047-10H0L3 |
| 2047-10H1-HZ1 | | 2047-10H1L0 | 2047-10H1L1 | 2047-10H1L2 | 2047-10H1L3 |
| 2047-10H1-HZ2 | | 2047-10H2L0 | 2047-10H2L1 | 2047-10H2L2 | 2047-10H2L3 |

### Example 6: Detection of the affinity of humanized antibodies

Experiments for measuring interactions between the humanized antibodies and human CD25-his were performed using BIAcore instrument X100. All the experiments were operated in HBS-EP (1×) buffer at 25 °C. An anti-histidine antibody was immobilized to the surface of CM5 chips by amino coupling method, and then human CD25-his at a concentration of 1 µg/ml was loaded and captured for 60 s. Starting at 50 nM, each solution containing an anti-CD25 antibody was 2-fold diluted to obtain serial dilutions of 6 concentrations in total. The dilutions were injected, and association was monitored for 180 s, and dissociation was monitored for 600 s. The chips were regenerated by injecting a glycine solution at pH 1.5. Kinetic data were analyzed using a 1:1 binding model. Specific results are shown in Table 22.

**Table 22: Affinity of the humanized antibodies detected**

| Antibody | ka (1/Ms) | kd (1/s) | KD (M) |
|---|---|---|---|
| 2046-18H1L1-m2 | 5.60E+05 | 1.37E-03 | 2.45E-09 |
| 2046-111H1L1-m2 | 6.52E+05 | 4.14E-03 | 6.35E-09 |
| 2046-36H1L1 | 3.96E+06 | 3.94E-03 | 9.94E-10 |
| 2047-10H2L2 | 4.61E+05 | 2.05E-03 | 4.46E-09 |
| 2047-3H1L2 | 3.48E+05 | 4.98E-04 | 1.43E-09 |
| 686 | 1.48E+06 | 1.67E-03 | 1.13E-09 |

### Example 7: Activity of humanized antibodies in in vitro inhibiting STATS phosphorylation in Treg cells stimulated by IL2

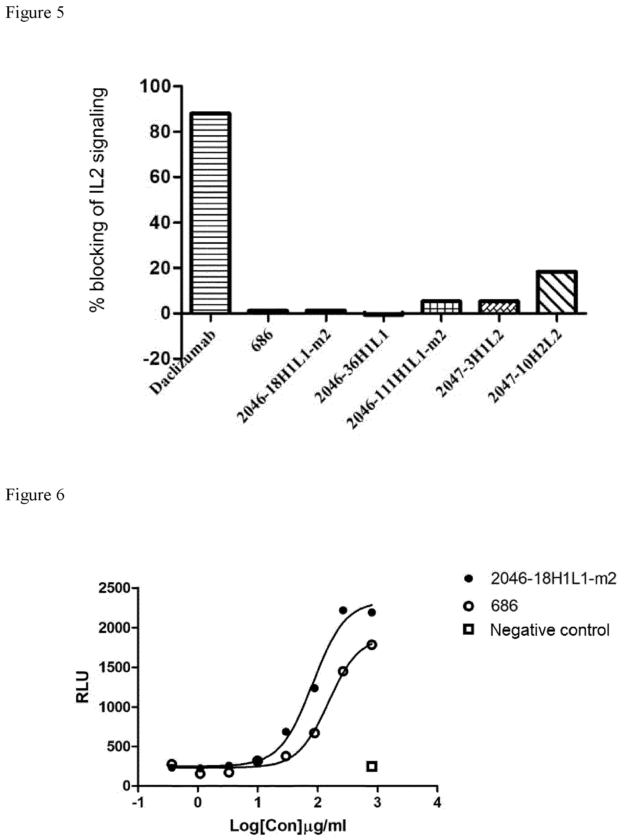

CD4⁺CD25⁺Treg cells were isolated from human PBMCs, and cultured in 1.5 ml EP tubes. Anti-CD25 antibodies (Daclizumab was used as a negative control, and 686 was used as a positive control) were diluted with RPMI-1640+10% FBS to obtain a concentration of 10 µg/ml. The antibodies were added into the EP tubes at 200 µl/tube and incubated for 30 min. Then the supernatants in the tubes were removed by centrifugation, and 200 µl of IL-2 at a concentration of 10 ng/ml was added into each tube which was then incubated for 15 min at 37 °C. After the incubation, the tubes were placed on an ice bath prepared in advance to stop the reaction; at the meantime, a centrifuge was set to 4 °C for precooling. Then the CD4⁺CD25⁺Treg cells in the tubes were centrifuged at 300×g, at 4 °C for 5 min, and the supernatants therein were discarded. After that, the cells were washed with PBS+3% FBS precooled on ice once, and fixed with 1.5% paraformaldehyde (precooled at 4 °C which was added into the cells at 100 µl/tube) for 20 min. Afterwards, the cells were washed with 900 µl of PBS +3% FBS added and were then centrifuged and supernatants were discarded. Next, ice-cold methanol precooled at -20 °C overnight was added at 100 µl/tube into the tubes, which were then placed on ice for performing permeabilization for 5 min. Then the fixed and permeabilized CD4⁺CD25⁺Treg cells were washed with 900 µl of PBS+3% FBS added and were then were centrifuged at 300×g for 5 min, and supernatants were discarded. The cells were washed once with 500 µl of PBS+3% FBS added again, and then were centrifuged at 300×g for 5 min. After that, the cells were resuspended in 100 µl of PBS, and Alexa Fluor647 Mouse Anti-Stat5 (pY694) antibody was added into the cells, at 5 µl/tube. The cells and the antibody were mixed well, and incubated for 30 min - 1 h at room temperature in dark. The supernatants were removed by centrifugation, and the cells were washed 2 times with PBS and resuspended in 200 µl of PBS. Then on-machine detection was performed. Results are shown in Figure 5. As can be seen from Figure 5, like 686, the obtained humanized antibodies are all non-IL2 blocking antibodies.

### Example 8: Activity of humanized antibodies in in vitro killing Treg cells

CD4⁺CD25⁺Treg cells were isolated from human PBMCs. Humanized antibodies were diluted in 96-well plates and added into 384-well white/clear bottom cell culture plates (Corning, cat. 3765) at 20 µl/well, which were then placed at 37 °C, 5% CO₂. ADCC effector cells (jurkat/CD16a/NFAT-luc cells) were counted, and an appropriate amount of the cells were centrifuged at 1500 rpm for 5 min, and then were resuspended in a culture medium to obtain a density of 2.4×10⁶ cells/ml. A suspension of Treg cells at a density of 4×10⁵ cells/ml was mixed with the suspension of ADCC effector cells at a density of 2.4×10⁶ cells/ml in a 1:1 volume ratio, so that in the obtained suspension, the density of Treg cells was 2×10⁵ cells/ml and the density of ADCC effector cells was 1.2×10⁶ cells/ml. The cells (E:T = 6:1) were added to the 384-well white/clear cell culture plates at 20 µl/well, to which the antibodies had been added, and incubated overnight (22-24 h) at 37 °C, 5% CO₂. After the incubation, Bio-Lite Luciferase Assay System was added into the plates at 40 µl/well, and chemiluminescence values were detected in a microplate reader. Specific results are shown in Table 23 and Figure 6. As can be seen, humanized antibody 2046-18H1L1-m2 have obviously better activity than 686 in killing Tregs in vitro, which was 1.85 times higher than that of 686.

**Table 23: Activity of a humanized antibody in killing Tregs in vitro and relative activity of the antibody compared with 686**

| Antibody | EC₅₀ (µg/mL) | Relative activity (%) |
|---|---|---|
| 686 | 152.60 | 100.00% |
| 2046-18H1L1-m2 | 82.58 | 184.79% |

### Example 9: Pharmacokinetics of humanized antibodies in wild-type mice

After an acclimation period, 30 mice were randomly divided into 6 groups, 5 mice in each. The mice were i.p. administrated according to the grouping, and time of administration was recorded. Blood of the mice in each group was sampled before administration (0 hr), and 4 hrs, 8 hrs, 24 hrs (1 d), 72 hrs (3 d), 120 hrs (5 d), 168 hrs (7 d), 240 hrs (10 d), 288 hrs (12 d), and 336 hrs (14 d) after administration, and sera were collected and stored at -60 to -80 °C.

Blood drug concentration for PK study in mouse was detected as follows:
(1) Coating: human CD25-his was diluted to 1 µg/mL with PBS (pH 7.2-7.4), added to 96-well ELSIA plates at 50 µL/well, which were then sealed with film. The plates were placed at 2-8 °C for 15-20 hours, and then washed with PBST (containing 0.05% (v/v) Tween-20, pH 7.2-7.4) for 3 times.
(2) Blocking and drying: the plates were blocked with N502 at 200 µL/well at room temperature for 1-2 hours. After the blocking buffer was aspirated off, the plates were dried in an incubator at 25 °C for >1 hr, and then used immediately, or sealed with film and stored at 2-8 °C.
(3) Preparing standards used for plotting standard curve and quality controls: an antibody sample was diluted to 1000 ng/mL using mouse plasma (EDTA-K), followed by 2-fold gradient dilution to 15 ng/mL (7 concentrations including 1000 ng/mL). Besides, the same antibody sample as that used for plotting standard curve was quantitatively diluted to concentrations of 1000 ng/mL, 100 ng/mL, and 20 ng/mL, respectively, which dilutions were then used as quality controls (QCs).
(4) Preparing samples to be detected: samples at different blood sampling time points were obtained and diluted with mouse plasma to a concentration within the concentration range used for plotting standard curve.
(5) Diluting the standards, quality controls and samples: the standards, quality controls and samples were diluted 10-fold with 0.1% casein (prepared with PBS, pH 6.2) (for example, 10 µL diluted with 90 µL) and added to the dried plates at 50 µL/well, 2 replicates per sample. The plates were sealed with film and incubated at room temperature for 2 hrs, and then washed with PBST (containing 0.05% (v/v) Tween-20, pH 7.2-7.4) for 3 times.
(6) Incubating with a secondary antibody: a goat anti-human IgG Fc-HRP was diluted 50000-fold with 10% goat serum, added to the plates at 50 µL/well, which were then sealed and incubated at room temperature for 1 hr. Afterwards, the plates were washed with PBST (containing 0.05% (v/v) Tween-20, pH 7.2-7.4) for 3 times.
(7) Color developing: TMB which had been warmed to room temperature was added to the plates at 50 µL/well, to develop color in dark for 20 min.
(8) Stopping reaction and reading OD values: 2 M sulfuric acid was added at 100 µL/well into the plates, which were then tapped to mix the sulfuric acid homogenously. The concentration of each sample tested was calculated using OD readings measured at 450 nm, with 650 nm as a reference.

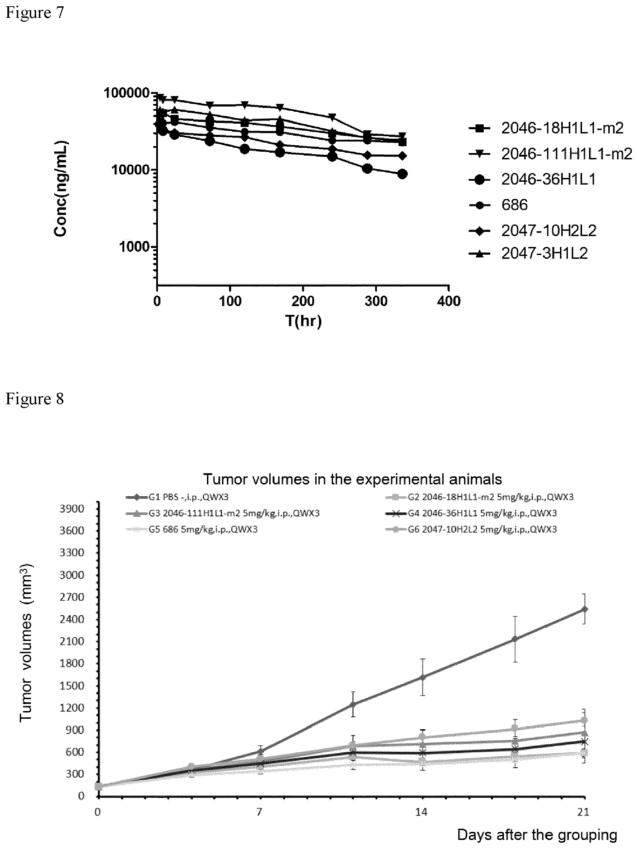

Experiment results showed that 6 antibodies had a half-life longer than 150 hrs. Specific results are shown in Figure 7.

### Example 10: Efficacy test of humanized antibodies in an animal model which were B-hIL2RA humanized mice engrafted with MC38 colon cancer cells

### 10.1: Analysis of tumor inhibition

MC38 cells resuspended in PBS were inoculated subcutaneously into the right flank region of B-hIL2RA humanized mice (Biocytogen) at a concentration of 5×10⁵ cells/0.1 mL, 0.1 mL per mouse. When the mean tumor volume reached 150±50 mm³, suitable mice were selected according to the tumor volume and body weight of the mice and evenly distributed into 6 experimental groups, with 6 mice in each group. Administration was started on the day of grouping (intraperitoneally injected at 5 mg/kg, once a week, 3 times in total). After the grouping, tumor volumes were measured two times per week using a calliper, and also were measured before the mice were euthanized. The long diameter and short diameter of each tumor were measured, and the tumor volume was calculated according to the following formula: tumor volume = 0.5 × long diameter × short diameter². The results showed that compared with the control group, the group administered with 2046-18H1L1-m2 and the group administered with 686 exhibited the best inhibitory effect, having a tumor growth inhibition rate above 80%; followed by the group administered with 2046-111H1L1-m2 and 2046-36H1L1, both having a tumor growth inhibition rate of approximately 70%. Specific results are shown in Figure 8.

### 10.2: Analysis of tumor-infiltrating lymphocytes (TILs)

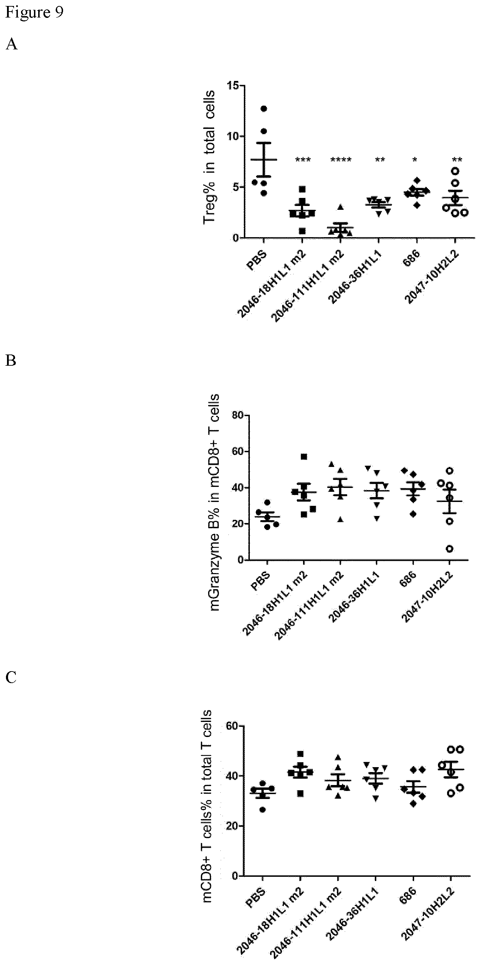

21 days after the grouping, the lymphocyte infiltration in the tumors of the mice was observed. The tumors were taken for analyzing TILs, and the results are shown in Figure 9 (in which, m means mouse). Compared with the control group (PBS), all the group administered with 2046-18H1L1-m2, the group administered with 2046-111H1L1-m2, the group administered with 2046-36H1L1, the group administered with 686, and the group administered with 2047-10H2L2 showed obviously less Tregs, which is consistent with the mechanism that the antibodies deplete Tregs. Further analysis showed that, the decline in the number of Tregs in the group administered with 2046-18H1L1-m2 and the group administered with 2046-111H1L1-m2 was more pronounced. In addition, compared with the control group, all the group administered with 2046-18H1L1-m2, the group administered with 2046-111H1L1-m2, the group administered with 2046-36H1L1, the group administered with 686, and the group administered with 2047-10H2L2 showed a certain increase in the number of CD8+ Teffs and Granzyme B+CD8+ Teffs, indicating that these non-IL2 blocking anti-CD25 antibodies are able to correct tumor microenvironment and increase the proportion and the killing ability of Teffs, in addition to depleting Tregs.

The above description of the embodiments of the present invention is not intended to limit the present invention, and those skilled in the art may make various changes and modifications to the present invention without departing from the spirit of the present invention, which should fall within the scope of the appended claims.

## Claims

1. An antibody molecule or antigen-binding fragment thereof comprising a heavy chain variable region (VH) and a light chain variable region (VL), wherein the heavy chain variable region (VH) and the light chain variable region (VL) comprise heavy chain CDRs and light chain CDRs selected from the group consisting of the following combinations of CDRs:
(1) H-CDR1, H-CDR2, and H-CDR3 as shown in SEQ ID NOs: 63, 64 and 65 respectively; and L-CDR1, L-CDR2, L-CDR3 as shown in SEQ ID NOs: 82, 83, and 84 respectively;
(2) H-CDR1, H-CDR2, and H-CDR3 as shown in SEQ ID NOs: 63, 64, and 65 respectively; and L-CDR1, L-CDR2, L-CDR3 as shown in SEQ ID NOs: 85, 83, and 84 respectively;
(3) H-CDR1, H-CDR2, and H-CDR3 as shown in SEQ ID NOs: 63, 66, and 65 respectively; and L-CDR1, L-CDR2, L-CDR3 as shown in SEQ ID NOs: 86, 83, and 84 respectively;
(4) H-CDR1, H-CDR2, and H-CDR3 as shown in SEQ ID NOs: 63, 66, and 65 respectively; and L-CDR1, L-CDR2, L-CDR3 as shown in SEQ ID NOs: 87, 83, and 84 respectively;
(5) H-CDR1, H-CDR2, and H-CDR3 as shown in SEQ ID NOs: 63, 67, and 68 respectively; and L-CDR1, L-CDR2, L-CDR3 as shown in SEQ ID NOs: 82, 83, and 88 respectively;
(6) H-CDR1, H-CDR2, and H-CDR3 as shown in SEQ ID NOs: 63, 67, and 68 respectively; and L-CDR1, L-CDR2, L-CDR3 as shown in SEQ ID NOs: 85, 83, and 88 respectively;
(7) H-CDR1, H-CDR2, and H-CDR3 as shown in SEQ ID NOs: 69, 70, and 71 respectively; and L-CDR1, L-CDR2, L-CDR3 as shown in SEQ ID NOs: 89, 90, and 91 respectively;
(8) H-CDR1, H-CDR2, and H-CDR3 as shown in SEQ ID NOs: 69, 70, and 71 respectively; and L-CDR1, L-CDR2, L-CDR3 as shown in SEQ ID NOs: 92, 90, and 91 respectively;
(9) H-CDR1, H-CDR2, and H-CDR3 as shown in SEQ ID NOs: 69, 70, and 71 respectively; and L-CDR1, L-CDR2, L-CDR3 as shown in SEQ ID NOs: 92, 93, and 91 respectively;
(10) H-CDR1, H-CDR2, and H-CDR3 as shown in SEQ ID NOs: 63, 72, and 68 respectively; and L-CDR1, L-CDR2, L-CDR3 as shown in SEQ ID NOs: 82, 83, and 84 respectively;
(11) H-CDR1, H-CDR2, and H-CDR3 as shown in SEQ ID NOs: 63, 72, and 68 respectively; and L-CDR1, L-CDR2, L-CDR3 as shown in SEQ ID NOs: 85, 83, and 84 respectively;
(12) H-CDR1, H-CDR2, and H-CDR3 as shown in SEQ ID NOs: 73, 74, and 68 respectively; and L-CDR1, L-CDR2, L-CDR3 as shown in SEQ ID NOs: 82, 83, and 84 respectively;
(13) H-CDR1, H-CDR2, and H-CDR3 as shown in SEQ ID NOs: 73, 74, and 68 respectively; and L-CDR1, L-CDR2, L-CDR3 as shown in SEQ ID NOs: 85, 83, and 84 respectively;
(14) H-CDR1, H-CDR2, and H-CDR3 as shown in SEQ ID NOs: 75, 76, and 77 respectively; and L-CDR1, L-CDR2, L-CDR3 as shown in SEQ ID NOs: 94, 95, and 96 respectively;
(15) H-CDR1, H-CDR2, and H-CDR3 as shown in SEQ ID NOs: 75, 76, and 77 respectively; and L-CDR1, L-CDR2, L-CDR3 as shown in SEQ ID NOs: 94, 95, and 97 respectively;
(16) H-CDR1, H-CDR2, and H-CDR3 as shown in SEQ ID NOs: 75, 76, and 77 respectively; and L-CDR1, L-CDR2, L-CDR3 as shown in SEQ ID NOs: 94, 95, and 98 respectively;
(17) H-CDR1, H-CDR2, and H-CDR3 as shown in SEQ ID NOs: 78, 79, and 80 respectively; and L-CDR1, L-CDR2, L-CDR3 as shown in SEQ ID NOs: 99, 100, and 101 respectively;
(18) H-CDR1, H-CDR2, and H-CDR3 as shown in SEQ ID NOs: 78, 81, and 80 respectively; and L-CDR1, L-CDR2, L-CDR3 as shown in SEQ ID NOs: 99, 100, and 101 respectively;
(19) H-CDR1, H-CDR2, and H-CDR3 as shown in SEQ ID NOs: 78, 79, and 80 respectively; and L-CDR1, L-CDR2, L-CDR3 as shown in SEQ ID NOs: 102, 100, and 101 respectively;
(20) H-CDR1, H-CDR2, and H-CDR3 as shown in SEQ ID NOs: 78, 81, and 80 respectively; and L-CDR1, L-CDR2, L-CDR3 as shown in SEQ ID NOs: 102, 100, and 101 respectively;
(21) H-CDR1, H-CDR2, and H-CDR3 as shown in SEQ ID NOs: 78, 79, and 80 respectively; and L-CDR1, L-CDR2, L-CDR3 as shown in SEQ ID NOs: 102, 103, and 101 respectively; and
(22) H-CDR1, H-CDR2, and H-CDR3 as shown in SEQ ID NOs: 78, 81, and 80 respectively; and L-CDR1, L-CDR2, L-CDR3 as shown in SEQ ID NOs: 102, 103, and 101 respectively.

2. The antibody molecule or antigen-binding fragment thereof according to claim 1, wherein the antibody molecule or antigen-binding fragment thereof is an antibody molecule or antigen-binding fragment thereof against CD25;
preferably, the CD25 is human CD25.

3. The antibody molecule or antigen-binding fragment thereof of according to claim 1 or 2, wherein the heavy chain variable region comprises an amino acid sequence as shown in any one of SEQ ID NOs: 1-25 or an amino acid sequence having at least 75% identity to the amino acid sequence as shown in any one of SEQ ID NOs: 1-25; and
the light chain variable region comprises an amino acid sequence as shown in any one of SEQ ID NOs: 26-62 or an amino acid sequence having at least 75% identity to the amino acid sequence as shown in any one of SEQ ID NOs: 26-62.

4. The antibody molecule or antigen-binding fragment thereof according to any one of claims 1 to 3, wherein the heavy chain variable region and the light chain variable region comprised in the antibody molecule or antigen-binding fragment thereof comprise amino acid sequences selected from the group consisting of the following combinations:
(1) an amino acid sequence as shown in SEQ ID NO: 1 or an amino acid sequence having at least 75% identity to the amino acid sequence as shown in SEQ ID NO: 1; and, an amino acid sequence as shown in SEQ ID NO: 26 or an amino acid sequence having at least 75% identity to the amino acid sequence as shown in SEQ ID NO: 26;
(2) an amino acid sequence as shown in SEQ ID NO: 2 or an amino acid sequence having at least 75% identity to the amino acid sequence as shown in SEQ ID NO: 2; and, an amino acid sequence as shown in SEQ ID NO: 27 or an amino acid sequence having at least 75% identity to the amino acid sequence as shown in SEQ ID NO: 27;
(3) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 27 or an amino acid sequence having at least 75% identity to the amino acid sequence as shown in SEQ ID NO: 27;
(4) an amino acid sequence as shown in SEQ ID NO: 2 or an amino acid sequence having at least 75% identity to the amino acid sequence as shown in SEQ ID NO: 2; and, an amino acid sequence as shown in SEQ ID NO: 28 or an amino acid sequence having at least 75% identity to the amino acid sequence as shown in SEQ ID NO: 28;
(5) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 28 or an amino acid sequence having at least 75% identity to the amino acid sequence as shown in SEQ ID NO: 28;
(6) an amino acid sequence as shown in SEQ ID NO: 2 or an amino acid sequence having at least 75% identity to the amino acid sequence as shown in SEQ ID NO: 2; and, an amino acid sequence as shown in SEQ ID NO: 29 or an amino acid sequence having at least 75% identity to the amino acid sequence as shown in SEQ ID NO: 29;
(7) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 29 or an amino acid sequence having at least 75% identity to the amino acid sequence as shown in SEQ ID NO: 29;
(8) an amino acid sequence as shown in SEQ ID NO: 4 or an amino acid sequence having at least 75% identity to the amino acid sequence as shown in SEQ ID NO: 4; and, an amino acid sequence as shown in SEQ ID NO: 30 or an amino acid sequence having at least 75% identity to the amino acid sequence as shown in SEQ ID NO: 30;
(9) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 31 or an amino acid sequence having at least 75% identity to the amino acid sequence as shown in SEQ ID NO: 31;
(10) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 31 or an amino acid sequence having at least 75% identity to the amino acid sequence as shown in SEQ ID NO: 31;
(11) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 32 or an amino acid sequence having at least 75% identity to the amino acid sequence as shown in SEQ ID NO: 32;
(12) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 32 or an amino acid sequence having at least 75% identity to the amino acid sequence as shown in SEQ ID NO: 32;
(13) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 33 or an amino acid sequence having at least 75% identity to the amino acid sequence as shown in SEQ ID NO: 33;
(14) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 33 or an amino acid sequence having at least 75% identity to the amino acid sequence as shown in SEQ ID NO: 33;
(15) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 34 or an amino acid sequence having at least 75% identity to the amino acid sequence as shown in SEQ ID NO: 34;
(16) an amino acid sequence as shown in SEQ ID NO: 7 or an amino acid sequence having at least 75% identity to the amino acid sequence as shown in SEQ ID NO: 7; and, an amino acid sequence as shown in SEQ ID NO: 35 or an amino acid sequence having at least 75% identity to the amino acid sequence as shown in SEQ ID NO: 35;
(17) an amino acid sequence as shown in SEQ ID NO: 8 or an amino acid sequence having at least 75% identity to the amino acid sequence as shown in SEQ ID NO: 8; and, an amino acid sequence as shown in SEQ ID NO: 36 or an amino acid sequence having at least 75% identity to the amino acid sequence as shown in SEQ ID NO: 36;
(18) an amino acid sequence as shown in SEQ ID NO: 9 or an amino acid sequence having at least 75% identity to the amino acid sequence as shown in SEQ ID NO: 9; and, an amino acid sequence as shown in SEQ ID NO: 36 or an amino acid sequence having at least 75% identity to the amino acid sequence as shown in SEQ ID NO: 36;
(19) an amino acid sequence as shown in SEQ ID NO: 8 or an amino acid sequence having at least 75% identity to the amino acid sequence as shown in SEQ ID NO: 8; and, an amino acid sequence as shown in SEQ ID NO: 37 or an amino acid sequence having at least 75% identity to the amino acid sequence as shown in SEQ ID NO: 37;
(20) an amino acid sequence as shown in SEQ ID NO: 9 or an amino acid sequence having at least 75% identity to the amino acid sequence as shown in SEQ ID NO: 9; and, an amino acid sequence as shown in SEQ ID NO: 37 or an amino acid sequence having at least 75% identity to the amino acid sequence as shown in SEQ ID NO: 37;
(21) an amino acid sequence as shown in SEQ ID NO: 8 or an amino acid sequence having at least 75% identity to the amino acid sequence as shown in SEQ ID NO: 8; and, an amino acid sequence as shown in SEQ ID NO: 38 or an amino acid sequence having at least 75% identity to the amino acid sequence as shown in SEQ ID NO: 38;
(22) an amino acid sequence as shown in SEQ ID NO: 9 or an amino acid sequence having at least 75% identity to the amino acid sequence as shown in SEQ ID NO: 9; and, an amino acid sequence as shown in SEQ ID NO: 38 or an amino acid sequence having at least 75% identity to the amino acid sequence as shown in SEQ ID NO: 38;
(23) an amino acid sequence as shown in SEQ ID NO: 10 or an amino acid sequence having at least 75% identity to the amino acid sequence as shown in SEQ ID NO: 10; and, an amino acid sequence as shown in SEQ ID NO: 39 or an amino acid sequence having at least 75% identity to the amino acid sequence as shown in SEQ ID NO: 39;
(24) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 40 or an amino acid sequence having at least 75% identity to the amino acid sequence as shown in SEQ ID NO: 40;
(25) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 40 or an amino acid sequence having at least 75% identity to the amino acid sequence as shown in SEQ ID NO: 40;
(26) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 41 or an amino acid sequence having at least 75% identity to the amino acid sequence as shown in SEQ ID NO: 41;
(27) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 41 or an amino acid sequence having at least 75% identity to the amino acid sequence as shown in SEQ ID NO: 41;
(28) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 42 or an amino acid sequence having at least 75% identity to the amino acid sequence as shown in SEQ ID NO: 42;
(29) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 42 or an amino acid sequence having at least 75% identity to the amino acid sequence as shown in SEQ ID NO: 42;
(30) an amino acid sequence as shown in SEQ ID NO: 11 or an amino acid sequence having at least 75% identity to the amino acid sequence as shown in SEQ ID NO: 11; and, an amino acid sequence as shown in SEQ ID NO: 43 or an amino acid sequence having at least 75% identity to the amino acid sequence as shown in SEQ ID NO: 43;
(31) an amino acid sequence as shown in SEQ ID NO: 12 or an amino acid sequence having at least 75% identity to the amino acid sequence as shown in SEQ ID NO: 12; and, an amino acid sequence as shown in SEQ ID NO: 43 or an amino acid sequence having at least 75% identity to the amino acid sequence as shown in SEQ ID NO: 43;
(32) an amino acid sequence as shown in SEQ ID NO: 13 or an amino acid sequence having at least 75% identity to the amino acid sequence as shown in SEQ ID NO: 13; and, an amino acid sequence as shown in SEQ ID NO: 44 or an amino acid sequence having at least 75% identity to the amino acid sequence as shown in SEQ ID NO: 44;
(33) an amino acid sequence as shown in SEQ ID NO: 14 or an amino acid sequence having at least 75% identity to the amino acid sequence as shown in SEQ ID NO: 14; and, an amino acid sequence as shown in SEQ ID NO: 45 or an amino acid sequence having at least 75% identity to the amino acid sequence as shown in SEQ ID NO: 45;
(34) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 45 or an amino acid sequence having at least 75% identity to the amino acid sequence as shown in SEQ ID NO: 45;
(35) an amino acid sequence as shown in SEQ ID NO: 14 or an amino acid sequence having at least 75% identity to the amino acid sequence as shown in SEQ ID NO: 14; and, an amino acid sequence as shown in SEQ ID NO: 46 or an amino acid sequence having at least 75% identity to the amino acid sequence as shown in SEQ ID NO: 46;
(36) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 46 or an amino acid sequence having at least 75% identity to the amino acid sequence as shown in SEQ ID NO: 46;
(37) an amino acid sequence as shown in SEQ ID NO: 14 or an amino acid sequence having at least 75% identity to the amino acid sequence as shown in SEQ ID NO: 14; and, an amino acid sequence as shown in SEQ ID NO: 47 or an amino acid sequence having at least 75% identity to the amino acid sequence as shown in SEQ ID NO: 47;
(38) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 47 or an amino acid sequence having at least 75% identity to the amino acid sequence as shown in SEQ ID NO: 47;
(39) an amino acid sequence as shown in SEQ ID NO: 16 or an amino acid sequence having at least 75% identity to the amino acid sequence as shown in SEQ ID NO: 16; and, an amino acid sequence as shown in SEQ ID NO: 48 or an amino acid sequence having at least 75% identity to the amino acid sequence as shown in SEQ ID NO: 48;
(40) an amino acid sequence as shown in SEQ ID NO: 17 or an amino acid sequence having at least 75% identity to the amino acid sequence as shown in SEQ ID NO: 17; and, an amino acid sequence as shown in SEQ ID NO: 49 or an amino acid sequence having at least 75% identity to the amino acid sequence as shown in SEQ ID NO: 49;
(41) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 49 or an amino acid sequence having at least 75% identity to the amino acid sequence as shown in SEQ ID NO: 49;
(42) an amino acid sequence as shown in SEQ ID NO: 17 or an amino acid sequence having at least 75% identity to the amino acid sequence as shown in SEQ ID NO: 17; and, an amino acid sequence as shown in SEQ ID NO: 50 or an amino acid sequence having at least 75% identity to the amino acid sequence as shown in SEQ ID NO: 50;
(43) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 50 or an amino acid sequence having at least 75% identity to the amino acid sequence as shown in SEQ ID NO: 50;
(44) an amino acid sequence as shown in SEQ ID NO: 17 or an amino acid sequence having at least 75% identity to the amino acid sequence as shown in SEQ ID NO: 17; and, an amino acid sequence as shown in SEQ ID NO: 51 or an amino acid sequence having at least 75% identity to the amino acid sequence as shown in SEQ ID NO: 51;
(45) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 51 or an amino acid sequence having at least 75% identity to the amino acid sequence as shown in SEQ ID NO: 51;
(46) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 52 or an amino acid sequence having at least 75% identity to the amino acid sequence as shown in SEQ ID NO: 52;
(47) an amino acid sequence as shown in SEQ ID NO: 19 or an amino acid sequence having at least 75% identity to the amino acid sequence as shown in SEQ ID NO: 19; and, an amino acid sequence as shown in SEQ ID NO: 53 or an amino acid sequence having at least 75% identity to the amino acid sequence as shown in SEQ ID NO: 53;
(48) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 54 or an amino acid sequence having at least 75% identity to the amino acid sequence as shown in SEQ ID NO: 54;
(49) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 54 or an amino acid sequence having at least 75% identity to the amino acid sequence as shown in SEQ ID NO: 54;
(50) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 55 or an amino acid sequence having at least 75% identity to the amino acid sequence as shown in SEQ ID NO: 55;
(51) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 55 or an amino acid sequence having at least 75% identity to the amino acid sequence as shown in SEQ ID NO: 55;
(52) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 56 or an amino acid sequence having at least 75% identity to the amino acid sequence as shown in SEQ ID NO: 56;
(53) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 56 or an amino acid sequence having at least 75% identity to the amino acid sequence as shown in SEQ ID NO: 56;
(54) an amino acid sequence as shown in SEQ ID NO: 20 or an amino acid sequence having at least 75% identity to the amino acid sequence as shown in SEQ ID NO: 20; and, an amino acid sequence as shown in SEQ ID NO: 57 or an amino acid sequence having at least 75% identity to the amino acid sequence as shown in SEQ ID NO: 57;
(55) an amino acid sequence as shown in SEQ ID NO: 21 or an amino acid sequence having at least 75% identity to the amino acid sequence as shown in SEQ ID NO: 21; and, an amino acid sequence as shown in SEQ ID NO: 57 or an amino acid sequence having at least 75% identity to the amino acid sequence as shown in SEQ ID NO: 57;
(56) an amino acid sequence as shown in SEQ ID NO: 22 or an amino acid sequence having at least 75% identity to the amino acid sequence as shown in SEQ ID NO: 22; and, an amino acid sequence as shown in SEQ ID NO: 58 or an amino acid sequence having at least 75% identity to the amino acid sequence as shown in SEQ ID NO: 58;
(57) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 59 or an amino acid sequence having at least 75% identity to the amino acid sequence as shown in SEQ ID NO: 59;
(58) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 59 or an amino acid sequence having at least 75% identity to the amino acid sequence as shown in SEQ ID NO: 59;
(59) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 59 or an amino acid sequence having at least 75% identity to the amino acid sequence as shown in SEQ ID NO: 59;
(60) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 60 or an amino acid sequence having at least 75% identity to the amino acid sequence as shown in SEQ ID NO: 60;
(61) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 60 or an amino acid sequence having at least 75% identity to the amino acid sequence as shown in SEQ ID NO: 60;
(62) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 60 or an amino acid sequence having at least 75% identity to the amino acid sequence as shown in SEQ ID NO: 60;
(63) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 61 or an amino acid sequence having at least 75% identity to the amino acid sequence as shown in SEQ ID NO: 61;
(64) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 61 or an amino acid sequence having at least 75% identity to the amino acid sequence as shown in SEQ ID NO: 61;
(65) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 61 or an amino acid sequence having at least 75% identity to the amino acid sequence as shown in SEQ ID NO: 61;
(66) an amino acid sequence as shown in SEQ ID NO: 23 or an amino acid sequence having at least 75% identity to the amino acid sequence as shown in SEQ ID NO: 23; and, an amino acid sequence as shown in SEQ ID NO: 62 or an amino acid sequence having at least 75% identity to the amino acid sequence as shown in SEQ ID NO: 62;
(67) an amino acid sequence as shown in SEQ ID NO: 24 or an amino acid sequence having at least 75% identity to the amino acid sequence as shown in SEQ ID NO: 24; and, an amino acid sequence as shown in SEQ ID NO: 62 or an amino acid sequence having at least 75% identity to the amino acid sequence as shown in SEQ ID NO: 62; and
(68) an amino acid sequence as shown in SEQ ID NO: 25 or an amino acid sequence having at least 75% identity to the amino acid sequence as shown in SEQ ID NO: 25; and, an amino acid sequence as shown in SEQ ID NO: 62 or an amino acid sequence having at least 75% identity to the amino acid sequence as shown in SEQ ID NO: 62.

5. The antibody molecule or antigen-binding fragment thereof according to any one of claims 1 to 4, wherein the antibody molecule is a murine antibody, a chimeric antibody, or a fully or partially humanized antibody; and the antigen-binding fragment is a half-antibody or a scFv, dsFv, (dsFv)₂, Fab, Fab', F(ab')₂, or Fv fragment of the antibody molecule;
preferably, the antibody molecule is a monoclonal antibody or a single chain antibody;
preferably, the antibody molecule or antigen-binding fragment thereof further comprises a human or murine constant region, preferably a human or murine heavy chain constant region (CH) and/or a light chain constant region (CL); preferably, the antibody molecule or antigen-binding fragment thereof comprises a heavy chain and a light chain;
more preferably, the antibody molecule or antigen-binding fragment thereof comprises a heavy chain constant region of an IgG, IgA, IgM, IgD, or IgE and/or a light chain constant region of a κ or λ type.

6. The antibody molecule or antigen-binding fragment thereof according to any one of claims 1 to 5, wherein the antibody molecule is a monoclonal antibody, preferably a chimeric or humanized monoclonal antibody;
preferably, the heavy chain constant region of the antibody molecule comprises an amino acid sequence as shown in SEQ ID NO: 104 or an amino acid sequence having at least 75% identity to the amino acid sequence as shown in SEQ ID NO: 104; and the light chain constant region of the antibody molecule comprises an amino acid sequence as shown in SEQ ID NO: 105 or an amino acid sequence having at least 75% identity to the amino acid sequence as shown in SEQ ID NO: 105;
preferably, the at least 75% identity is any percent identity greater than or equal to 75%, such as at least 80%, preferably at least 85%, more preferably at least 90%, further preferably at least 91%, 92%, 93%, 94%, 95%, 96%, 97%, 98%, or even 99% identity.

7. A nucleic acid molecule comprising a nucleotide sequence encoding a heavy chain CDR, a light chain CDR, a heavy chain variable region, a light chain variable region, a heavy chain or a light chain comprised in the antibody molecule or antigen-binding fragment thereof according to any one of claims 1 to 6.

8. A vector comprising the nucleic acid molecule according to claim 7.

9. A host cell comprising the nucleic acid molecule according to claim 7 and/or the vector according to claim 8, or transformed or transfected with the nucleic acid molecule according to claim 7 and/or the vector according to claim 8.

10. A composition comprising the antibody molecule or antigen-binding fragment thereof according to any one of claims 1 to 6, the nucleic acid molecule according to claim 7, the vector according to claim 8, and/or the host cell according to claim 9;
preferably, the composition is a pharmaceutical composition, optionally comprising a pharmaceutically acceptable carrier, auxiliary material, or excipient.

11. Use of the antibody molecule or antigen-binding fragment thereof according to any one of claims 1 to 6, the nucleic acid molecule according to claim 7, the vector according to claim 8, the host cell according to claim 9, and/or the composition according to claim 10 in the manufacture of a medicament for preventing, treating, and/or ameliorating a solid tumor or a hematological tumor.

12. A method for preventing, treating and/or ameliorating a disease, comprising administering to a subject in need thereof a therapeutically effective amount of the antibody molecule or antigen-binding fragment thereof according to any one of claims 1 to 6, the nucleic acid molecule according to claim 7, the vector according to claim 8, the host cell according to claim 9, and/or the composition according to claim 10, and optionally an additional drug or means; preferably, the disease is a solid tumor or a hematological tumor;
preferably, the subject is a mammal; preferably, the subject is a human.

13. A method for detecting or diagnosing a disease, comprising contacting the antibody molecule or antigen-binding fragment thereof according to any one of claims 1 to 6, the nucleic acid molecule according to claim 7, the vector according to claim 8, the host cell according to claim 9, and/or the composition according to claim 10 with a sample from a subject; preferably, the disease is a solid tumor or a hematological tumor;
preferably, the subject is a mammal; preferably, the subject is a human.

14. A kit comprising the antibody molecule or antigen-binding fragment thereof according to any one of claims 1 to 6, the nucleic acid molecule according to claim 7, the vector according to claim 8, the host cell according to claim 9, and/or the composition according to claim 10.
